# EUROPEAN PATENT APPLICATION

(11) **EP 4 596 531 A1**
(43) Date of publication of application: **06.08.2025**
(21) Application number: 25152443.5
(22) Date of filing: 17.01.2025
(51) Int. Cl.: C07C 235/10, A61K 9/1272, A61K 9/51, C07D 231/40, C07D 295/13, C07D 213/40

(54) **IONIZABLE LIPID, PREPARATION METHOD THEREOF, AND COMPOSITION FOR PREPARING LIPID NANOPARTICLE**

(30) Priority: 30.01.2024 US 202463626587 P; 19.11.2024 TW 113144353
(71) Applicant: Acer Incorporated, Hsichih, New Taipei City 221 (TW)
(72) Inventor: HSU, Shao-Chien, 221 New Taipei City (TW); CHEN, Po-Ming, 221 New Taipei City (TW); CHOU, Yi-Te, 221 New Taipei City (TW); LIN, Jhe-Jhih, 221 New Taipei City (TW); CHIU, Chih-Wei, 221 New Taipei City (TW)
(74) Representative: 2K Patentanwälte Blasberg Kewitz & Reichel

(57) **Abstract**

An ionizable lipid having a structure represented by the following formula (I): 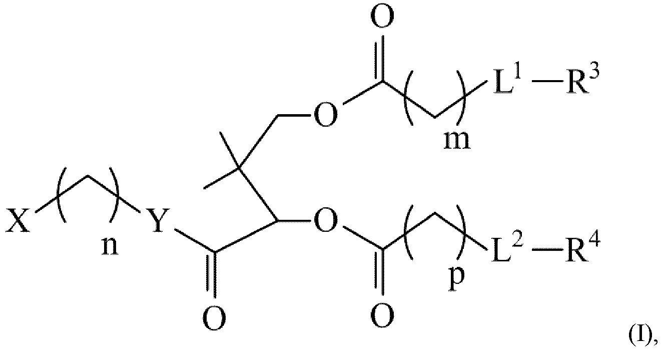 wherein Y is independently selected from a group consisting of -NH-, -O-, -S-, and a single bond; X is independently selected from -NR¹R² or a nitrogen-containing heteroaryl group; L¹ and L² are each independently selected from a group consisting of a C₁-C₁₀ alkylene group, a C₂-C₁₀ alkenylene group, 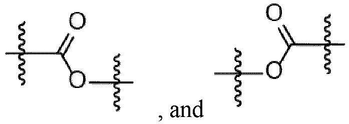 ; R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group; R³ is a C₅-C₃₀ alkyl group; R⁴ is a C₅-C₃₀ alkyl group; n is an integer selected from 1 to 10; and m and p are each independently an integer selected from 1 to 20.

## Description

### CROSS REFERENCE TO RELATED APPLICATIONS

This application claims the benefit of U.S. Provisional Application No. 63/626,587, filed January 30, 2024, and claims priority of Taiwan Patent Application No. 113144353, filed on November 19, 2024, the entirety of which is incorporated by reference.

### BACKGROUND OF THE INVENTION

### Field of the Invention

The present invention relates to a lipid, a preparation method thereof, and a composition including the same, and in particular it relates to an ionizable lipid, a preparation method thereof, and a composition for preparing a lipid nanoparticle.

### Description of the Related Art

Lipid nanoparticles (LNP) are a type of Lipid-based nanocarrier. Lipid nanoparticles can penetrate cell membranes and allow vaccines (or drugs) to enter target cells and are highly promising vehicles for vaccine (or drug) delivery.

Lipid nanoparticles may include ionizable lipids. Ionizable lipids can enhance the ability of the lipid nanoparticles to bind to antibodies or peptides, and can enhance the targeting ability by the specificity of the antibodies to the peptides. In addition, the ionizable lipids are also useful for the self-assembly of nanoparticles and ribonucleic acid (RNA), helping target cells to absorb vaccines (or drugs).

### BRIEF SUMMARY OF THE INVENTION

Some embodiments of the present disclosure provide an ionizable lipid having a structure represented by the following formula (I): 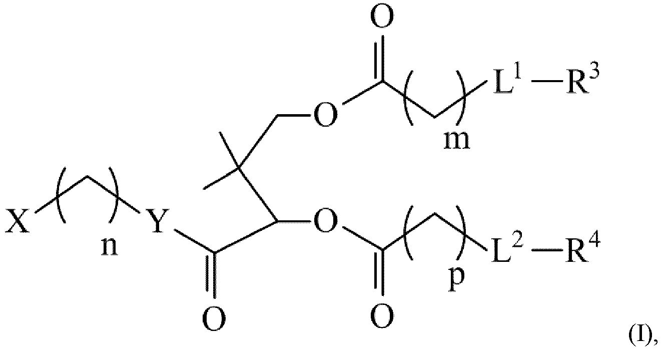
wherein Y is independently selected from a group consisting of -NH-, -O-, -S-, and a single bond;
X is independently selected from -NR¹R² or a nitrogen-containing heteroaryl group;
L¹ and L² are each independently selected from a group consisting of a C₁-C₁₀ alkylene group, a C₂-C₁₀ alkenylene group, 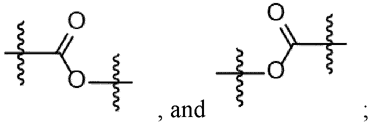
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each independently an integer selected from 1 to 20.

Some embodiments of the present disclosure provide a composition for preparing a lipid nanoparticle. The composition includes 30 to 75 parts by weight of an ionizable lipid; 5 to 20 parts by weight of a phospholipid compound; 19.5 to 60 parts by weight of a sterol compound; and 0.5 to 5 parts by weight of a polyethylene glycol lipid, wherein the ionizable lipid has a structure represented by the following formula (I): 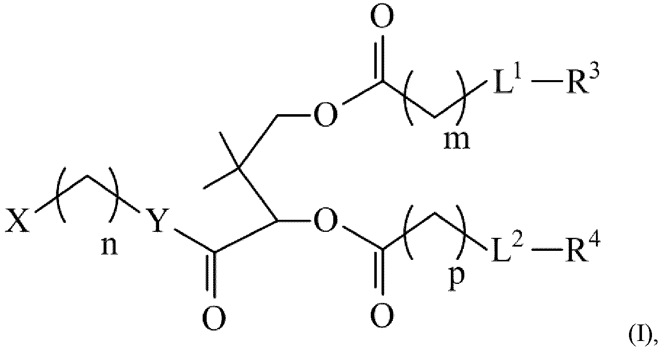
where Y is independently selected from a group consisting of -NH-, -O-, -S-, and a single bond;
X is independently selected from -NR¹R² or a nitrogen-containing heteroaryl group;
L¹ and L² are each independently selected from a group consisting of a C₁-C₁₀ alkylene group, a C₂-C₁₀ alkenylene group, 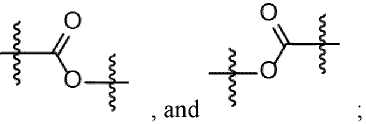
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each an integer independently selected from 1 to 20.

Some embodiments of the present disclosure provide a preparation method of an ionizable lipid. The method includes forming an intermediate product from a diamine compound and a pan-lactone; mixing a carboxylic acid compound with the intermediate product to obtain a mixture; and esterifying the mixture to obtain the ionizable lipid, wherein the ionizable lipid has a structure represented by the following formula (II): 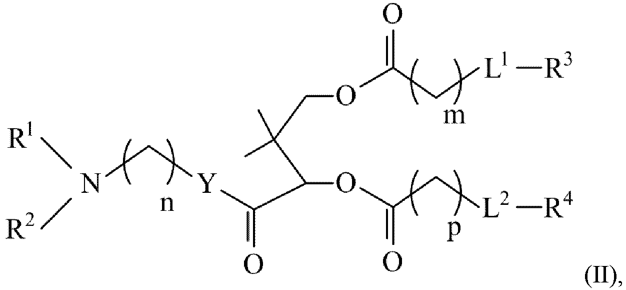
where Y is independently selected from a group consisting of -NH-, -O-, -S-, and a single bond;
L¹ and L² are each independently selected from a group consisting of a C₁-C₁₀ alkylene group, a C₂-C₁₀ alkenylene group, 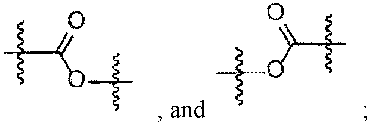
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each independently an integer selected from 1 to 20.

### BRIEF DESCRIPTION OF THE DRAWINGS

The present disclosure can be more fully understood by reading the subsequent detailed description and examples with references made to the accompanying drawings, wherein:
FIG. 1 is a flowchart of a preparation method of an ionizable lipid according to some embodiments of the present disclosure;
FIG. 2 is a graph of luciferase activity of HEK293 cell lines treated with a lipid nanoparticle agent prepared from a composition for preparing lipid nanoparticle according to embodiments and comparative examples of the present disclosure;
FIG. 3 is a cell viability graph of HEK293 cell lines treated with a lipid nanoparticle agent prepared from a composition for preparing lipid nanoparticle according to embodiments and comparative examples of the present disclosure;
FIG. 4 is a cell viability graph of HEK293 cell lines treated with a lipid nanoparticle agent prepared from a composition for preparing lipid nanoparticle according to embodiments and comparative examples of the present disclosure;
FIG. 5 is a graph of luciferase activity of HEK293 cell lines treated with a lipid nanoparticle agent prepared from a composition for preparing lipid nanoparticle according to embodiments and comparative examples of the present disclosure;
FIG. 6 is a cell viability graph of bone marrow-derived dendritic cells (BMDC) treated with a lipid nanoparticle agent prepared from a composition for preparing lipid nanoparticle according to embodiments and comparative examples of the present disclosure; and
FIG. 7 is a graph of luciferase activity of bone marrow-derived dendritic cells (BMDC) treated with a lipid nanoparticle agent prepared from a composition for preparing lipid nanoparticle according to embodiments and comparative examples of the present disclosure.

### DETAILED DESCRIPTION OF THE INVENTION

It will be further understood that the terms "comprises," "comprising," "includes" and/or "including," when used herein, specify the presence of stated features, integers, steps, operations, elements, components and/or groups thereof, but do not preclude the presence or addition of one or more other features, integers, steps, operations, elements, components and/or groups thereof. As used herein, the singular forms "a," "an" and "the" are intended to include the plural forms as well, unless the context clearly indicates otherwise.

It will be understood that, the term "about", "approximate", "rough" as used herein usually indicates a value of a given value or range that varies within 20%, preferably within 10%, and preferably within 5%, or within 3%, or within 2%, or within 1%, or within 0.5%. The value given here are approximate value, i.e., "about", "approximate", or "rough" may be implied without specifying "about", "approximate", or "rough". It will be further understood that the values indicated in herein may include the said values as well as deviation values that are within an acceptable deviation range for people having general knowledge in art. It will be understood that the expression "a to b" used herein to indicate a specific range of values is defined as "≧a and ≦b".

Unless otherwise defined, all terms (including technical and scientific terms) used herein have the same meaning as commonly understood by a person skilled in the art to which the invention pertains. It will be further understood that terms, such as those defined in commonly used dictionaries, should be interpreted as having a meaning consistent with the relevant technology and the context or background of this disclosure and will not be interpreted in an idealized or overly formal sense unless expressly so defined herein. Descriptions of known functions and constructions that may unnecessarily obscure the present disclosure will be omitted below.

The term "C₁-C₁₀ hydrocarbyl group" in the present disclosure refers to a linear, branched or cyclic, saturated or unsaturated group having 1 to 10 carbon atoms. The C₁-C₁₀ hydrocarbyl group of the present disclosure may include a C₁-C₁₀ alkyl group, a C₁-C₁₀ alkenyl group, a C₁-C₁₀ alkynyl group, and/or a combination thereof. The term "C₁-C₁₀ alkyl group" in the present disclosure refers to a linear, branched, or cyclic aliphatic hydrocarbon monovalent group having 1 to 10 carbon atoms in the main carbon chain thereof. Similarly, the term "C₅-C₃₀ alkyl group" in the present disclosure refers to a linear, branched, or cyclic aliphatic hydrocarbon monovalent group having 5 to 30 carbon atoms in the main carbon chain thereof. The term "C₁-C₁₀ alkenyl group" in the present disclosure refers to a linear, branched, or cyclic aliphatic hydrocarbon monovalent group having 1 to 10 carbon atoms and at least one carbon-carbon double bond in the main carbon chain thereof. The term "C₁-C₁₀ alkynyl group" in the present disclosure refers to a linear, branched, or cyclic aliphatic hydrocarbon monovalent group having 1 to 10 carbon atoms and at least one carbon-carbon triple bond in the main carbon chain thereof. The term "alkyl group" in the present disclosure includes a linear, branched or cyclic alkyl group. The term "alkenyl group" in the present disclosure includes a linear, branched or cyclic alkenyl group. The term "alkynyl group" in the present disclosure includes a linear, branched or cyclic alkynyl group. The C₁-C₁₀ hydrocarbyl group, the C₁-C₁₀ alkyl group, the C₅-C₃₀ alkyl group, the C₁-C₁₀ alkenyl group, and the C₁-C₁₀ alkynyl group of the present disclosure may be a substituted or unsubstituted C₁-C₁₀ hydrocarbyl group, a substituted or unsubstituted C₁-C₁₀ alkyl group, a substituted or unsubstituted C₅-C₃₀ alkyl group, a substituted or unsubstituted C₁-C₁₀ alkenyl group, and a substituted or unsubstituted C₁-C₁₀ alkynyl group.

Examples of the C₁-C₁₀ hydrocarbyl group in the present disclosure include, but are not limited to, a methyl group, an ethyl group, a vinyl group, an ethenyl group, an ethynyl group, a propyl group, a cyclopropyl group, a propenyl group, a propynyl group, an isobutyl group, a sec-butyl group, a tert-butyl group, a n-butenyl group, an isobutenyl group, a 1-butynyl group, a cyclohexyl group, a cyclohexenyl group, a cyclohexynyl group, an isopentyl group, a heptadienyl group, an isoprenyl group, and a cyclobutenyl group.

The term "C₁-C₁₀ alkylene group" in the present disclosure refers to a linear, branched or cyclic aliphatic hydrocarbon divalent group having 1 to 10 carbon atoms in the main carbon chain thereof. The C₁-C₁₀ alkylene group of the present disclosure may be a substituted or unsubstituted C₁-C₁₀ alkylene group. Non-limiting examples of the C₁-C₁₀ alkylene group include, but are not limited to, a methylene group, an ethylene group, a propylene group, an isobutylene group, a sec-butylene group, a tert-butylene group, a hexylene group, a cyclohexylene group, and a cyclooctylene group.

The term "C₂-C₁₀ alkenylene group" in the present disclosure refers to a linear, branched or cyclic aliphatic hydrocarbon divalent group having 2 to 10 carbon atoms and at least one carbon-carbon double bond in the main carbon chain thereof. The term "C₂-C₁₀ alkadienylene group" of the present disclosure refers to a linear, branched or cyclic aliphatic hydrocarbon divalent group having 2 to 10 carbon atoms and two carbon-carbon double bonds on the main carbon chain thereof. The C₂-C₁₀ alkenylene group of the present disclosure may be a substituted or unsubstituted C₂-C₁₀ alkenylene group.

Non-limiting examples of the C₂-C₁₀ alkenylene group in the present disclosure include, but are not limited to, an ethenylene group, a propenylene group, a butenylene group, a butadienylene group, a pentenylene group, an isopentenylene group, an isopentadienylene group, a hexenylene group, a cyclohexenylene group, a cyclohexadienylene group, a heptenylene group, a heptadienylene group, a cyclooctenylene group, a cyclooctadienylene group, a cyclooctatrienylene group, and a cyclooctatetraenylene group. In some embodiments, a non-limiting example of the C₂-C₁₀ alkenylene group includes 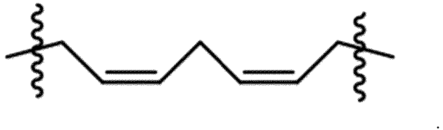 Non-limiting examples of the C₂-C₁₀ alkadienylene group in the present disclosure include, but are not limited to, a butadienylene group, an isopentadienylene group, a cyclohexadienylene group, a heptadienylene group, and a cyclooctadienylene group. In some embodiments, a non-limiting example of the C₂-C₁₀ alkadienylene group includes

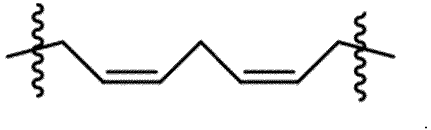

The term "C₁-C₁₀ heterohydrocarbyl group" in the present disclosure refers to a group including a heteroatom and a C₁-C₁₀ hydrocarbyl group, wherein the heteroatom may may be selected from a group consisting of N, P, O, and S. In some embodiments, the C₁-C₁₀ heterohydrocarbyl group may include a -NR, -PR, -OR, or -SR group, wherein R is the C₁-C₁₀ hydrocarbyl group. The term "C₁-C₁₀ heterohydrocarbyl group " in the present disclosure may be a substituted or unsubstituted C₁-C₁₀ heterohydrocarbyl group.

The term "heterocycloalkyl group" in the present disclosure refers to a cyclic aliphatic hydrocarbon monovalent group comprising a heteroatom as a ring-forming atom, wherein the heteroatom may be selected from a group consisting of N, P, O, and S. The heterocycloalkyl group may be a substituted or unsubstituted heterocycloalkyl group.

The term "C₆-C₂₀ aryl group" in the present disclosure refers to a monovalent group containing a carbocyclic aromatic system having 6 to 20 carbon atoms. The C₆-C₂₀ aryl group in the present disclosure may be a substituted or unsubstituted C₆-C₂₀ aryl group. Non-limiting examples of the C₆-C₂₀ aryl group include, but are not limited to, a phenyl group, a naphthyl group, an anthracenyl group, and a phenanthreyl group. The term "C₁-C₂₀ heteroaryl group" in the present disclosure refers to a monovalent group containing a carbocyclic aromatic system having 1 to 20 carbon atoms and a heteroatom as a ring-forming atom. The C₁-C₂₀ heteroaryl group of the present disclosure may be a substituted or unsubstituted C₁-C₂₀ heteroaryl group. Non-limiting examples of the C₁-C₂₀ heteroaryl group include, but are not limited to, a pyridinyl group, a diazolyl group, a triazolyl group, a pyranyl group, a diazinyl group, a thienyl group, a furanyl group, a pyrrolyl group, an imidazolyl group, and a pyrazolyl group. The term "nitrogen-containing heteroaryl group" in the present disclosure refers to a monovalent group containing a carbocyclic aromatic system having a nitrogen atom as a ring-forming atom.

The substituted C₁-C₁₀ hydrocarbyl group, the substituted C₁-C₁₀ alkyl group, the substituted C₅-C₃₀ alkyl group, the substituted C₁-C₁₀ alkenyl group, the substituted C₁-C₁₀ alkynyl group, the substituted C₁-C₁₀ alkylene group, the substituted C₂-C₁₀ alkenylene group, the substituted C₁-C₁₀ heterohydrocarbyl group, the substituted heterocycloalkyl group, the substituted C₆-C₂₀ aryl group, and the substituted C₁-C₂₀ heteroaryl group in the present disclosure refer to the C₁-C₁₀ hydrocarbyl group, the C₁-C₁₀ alkyl group, the C₅-C₃₀ alkyl group, the C₁-C₁₀ alkenyl group, the C₁-C₁₀ alkynyl group, the C₁-C₁₀ alkylene group, the C₂-C₁₀ alkenylene group, the C₁-C₁₀ heterohydrocarbyl group, the heterocycloalkyl group, the C₆-C₂₀ aryl group, and the C₁-C₂₀ heteroaryl group in which at least one of the hydrogen atoms thereof is substituted by a substituent. Examples of the substituent include, but are not limited to, a deuterium atom, -F, -Cl, -Br, -I, a hydroxyl group, a C₂-C₂₀ alkylether group (-C₁-C₁₀ alkyl-O-C₁-C₁₀ alkyl group), and a C₁-C₁₀ alkoxy group ( -O-C₁-C₁₀ alkyl group or - C₁-C₁₀ alkyl-O).

Some embodiments of the present disclosure provide an ionizable lipid suitable for use in the preparation of a lipid nanoparticle for use as a lipid nanoparticle carrier. The ionizable lipid has a structure represented by the following formula (I): 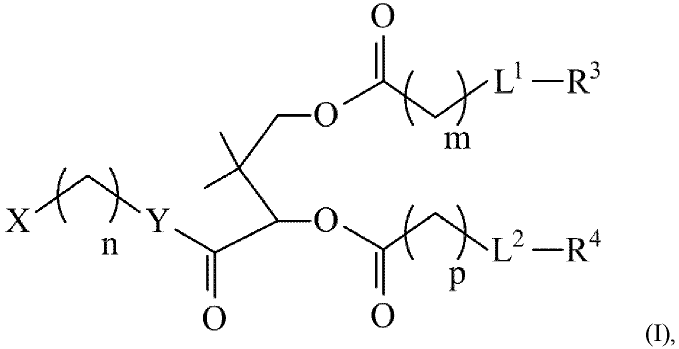
where Y is independently selected from a group consisting of -NH-, -O-, -S-, and a single bond;
X is independently selected from -NR¹R² or a nitrogen-containing heteroaryl group;
L¹ and L² are each independently selected from a group consisting of a C₁-C₁₀ alkylene group, a C₂-C₁₀ alkenylene group, 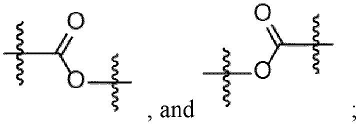
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each an integer independently selected from 1 to 20.

In some embodiments, Y may be -NH-, -O-, or a single bond. In some embodiments, Y may be -NH-.

In some embodiments, n may be an integer selected from 1 to 8, 1 to 5, 1 to 3, or 2 to 4. In some embodiments, n may be selected from 2 or 3. In some embodiments, n may be 2.

In some embodiments, m and p may be each an integer independently selected from 5 to 20, 6 to 20, 1 to 10, 4 to 10, 5 to 10, 6 to 10, 4 to 8, 5 to 8, or 6 to 8.

In some embodiments, R³ and R⁴ may be a C₅-C₃₀ branched alkyl group or a C₅-C₃₀ linear alkyl group. In some embodiments, R³ and R⁴ may be a C₈-C₂₅ branched alkyl group or a C₅-C₁₅ linear alkyl group. In some embodiments, R³ and R⁴ may be a C₁₀-C₂₄ branched alkyl group or a C₅-C₁₀ linear alkyl group. R³ and R⁴ may be the same or different from each other. For example, in some embodiments, R³ may be a C₅-C₃₀ branched alkyl group and R⁴ may be a C₅-C₃₀ linear alkyl group. In some embodiments, R³ may be a C₁₂ branched alkyl group and R⁴ may be a C₁₇ branched alkyl group. In some embodiments, R³ and R⁴ may both be C₁₂ branched alkyl groups, C₁₆ branched alkyl groups, or C₂₄ branched alkyl groups, but the present disclosure is not limited thereto.

L¹ and L² in formula (I) may be the same as or different from each other. For example, in some embodiments, L¹ and L² may be both 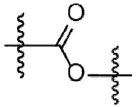 . In other some embodiments, L² may be 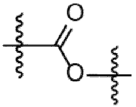 and L¹ may be selected from a group consisting of a C₁-C₁₀ alkylene group, a C₂-C₁₀ alkenylene group, and

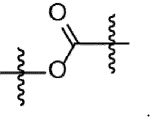

In the embodiment that L² is 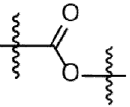 , the ionizable lipid of the present disclosure may have a structure represented by the following formula (I-1), wherein X, Y, L¹ , R³, R⁴ , n, m, and p are defined as described above:

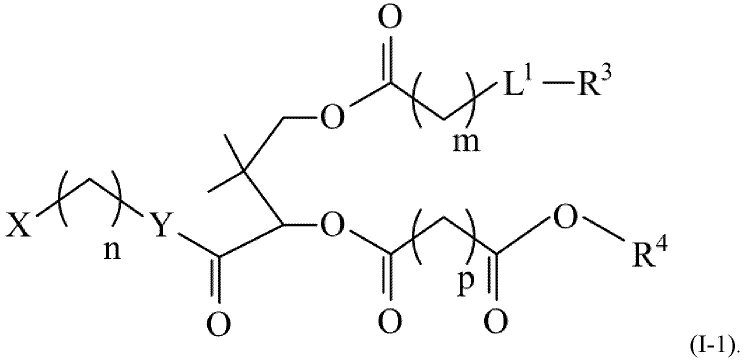

In this embodiment, L¹ may be independently selected from a group consisting of a C₁-C₁₀ alkylene group, a C₂-C₁₀ alkenylene group, 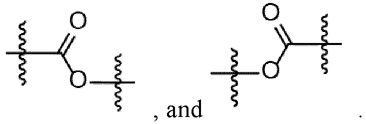 In some embodiments, L¹ may be a C₂-C₁₀ alkenylene group.

In some embodiments, L¹ in formula (I-1) may be a C₂-C₁₀ alkenylene group, R³ may be a C₅-C₃₀ linear alkyl group or a C₅-C₃₀ branched alkyl group, and R⁴ may be a C₅-C₃₀ branched alkyl group. In some embodiments, L¹ in formula (I-1) may be a C₂-C₁₀ alkenylene group, R³ may be a C₅-C₃₀ linear alkyl group, and R⁴ may be a C₅-C₃₀ branched alkyl group. In some embodiments, L¹ in formula (I-1) may be a C₃-C₈ alkenylene group, R³ may be a C₅-C₁₅ linear alkyl group, and R⁴ may be a C₈-C₂₅ branched alkyl group, but the present disclosure is not limited thereto.

In the embodiment that L¹ and L² in formula (I) are both 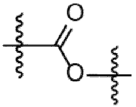 , the ionizable lipid of the present disclosure may have a structure represented by the following formula (I-2), wherein X, Y, R³ , R⁴ , n, m, and p are defined as described above:

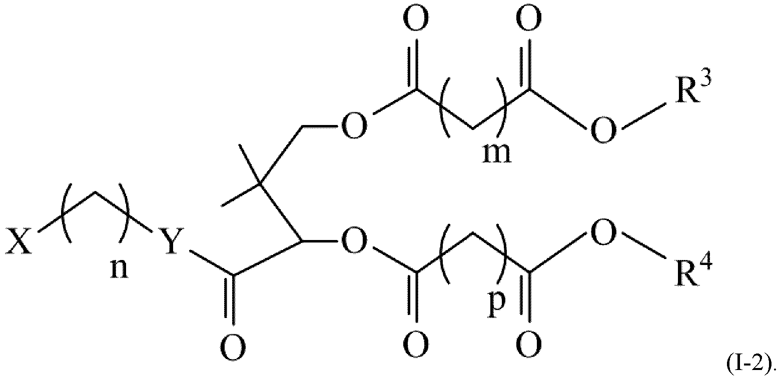

In some embodiments, R³ and R⁴ in formula (I-2) may each independently be a C₅-C₃₀ branched alkyl group. In some embodiments, R³ and R⁴ in formula (I-2) may each independently be a C₈-C₂₅ branched alkyl group, but the present disclosure is not limited thereto.

In some embodiments, L¹ and L² in formula (I) may each independently be a C₂-C₁₀ alkenylene group. In some embodiments, L¹ and L² may each independently be a C₂-C₁₀ alkadienylene group. In some embodiments, L¹ may be a propenylene group and L² may be a heptadienylene group, but the present disclosure is not limited thereto. In some embodiments, L¹ and L² may both be heptadienylene groups.

In some embodiments, X in formula (I) may be a nitrogen-containing heteroaryl group. In some embodiments, X in formula (I) may be a pyridyl group, but the present disclosure is not limited thereto. In some embodiments, X in formula (I) may be -NR¹R². In the embodiments that X in formula (I) is -NR¹R², the ionizable lipid of the present disclosure may have a structure represented by the following formula (II), wherein Y, L¹, L², R¹, R², R³, R⁴, n, m, and p are defined as above:

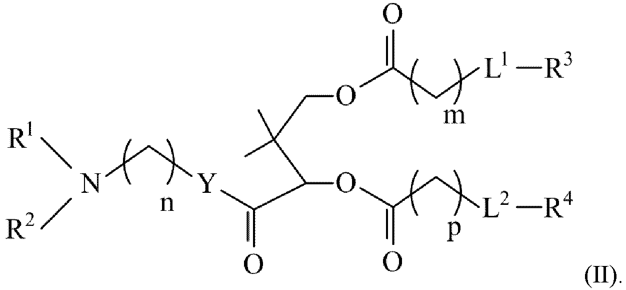

L² in formula (II) may be 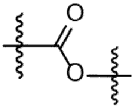 . In this embodiment, the ionizable lipid of the present disclosure may have a structure represented by the following formula (II-1), wherein Y, L¹, R¹, R², R³, R⁴, n, m, and p are defined as described above:

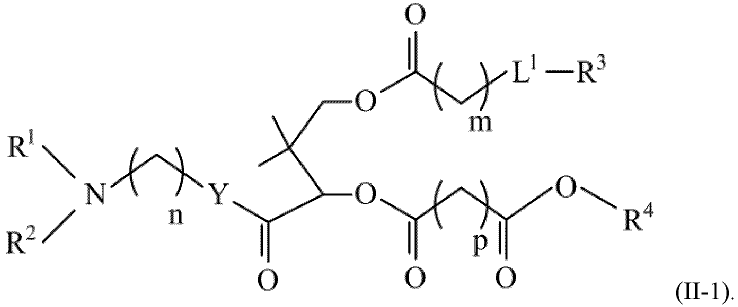

In this embodiment, L¹ may each be independently selected from a group consisting of a C₁-C₁₀ alkylene group, a C₂-C₁₀ alkenylene group, 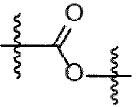 , and

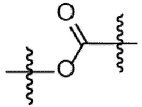

In the embodiment that L¹ and L² in formula (II) are both 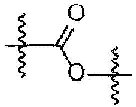 , the ionizable lipid of the present disclosure may have a structure represented by the following formula (II-2), wherein Y, R¹, R², R³, R⁴, n, m, and p are defined as described above:

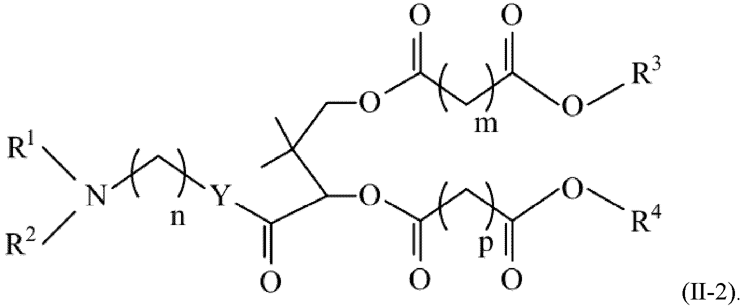

In some embodiments, R¹ and R² in formula (II), (II-1), or (II-2) may each be H, a substituted or unsubstituted C₁-C₁₀ hydrocarbyl group, or a substituted or unsubstituted C₁-C₁₀ heterohydrocarbyl group. In some embodiments, R¹ and R² may each be H, a substituted or unsubstituted C₁-C₁₀ alkyl group, or a substituted or unsubstituted C₁-C₁₀ heteroalkyl group.

In the embodiments that R¹ and R² in formula (II), (II-1), or (II-2) are substituted or unsubstituted C₁-C₁₀ hydrocarbyl groups, or substituted or unsubstituted C₁-C₁₀ heterohydrocarbyl groups, N and R¹ and R² may be bonded to each other to form a heterocycloalkyl group or a heteroaryl group. In some embodiments, the N bonded to R¹ and R² to form a heterocycloalkyl group or a heteroaryl group may be the only heteroatom in the formed heterocycloalkyl group or heteroaryl group, but the present disclosure is not limited thereto. In some embodiments, the formed heterocycloalkyl group or heteroaryl group may include at least one other heteroatom, and the at least one other heteroatom may be independently selected from N, O, P, or S. For example, in some embodiments, N and R¹ and R² may be bonded to each other to form a tetrahydrd-1,4-oxazine or a 1,4-diazacyclohexane. However, the present disclosure is not limited thereto.

In some embodiments, the ionizable lipid of the present disclosure may have a structure as shown below:

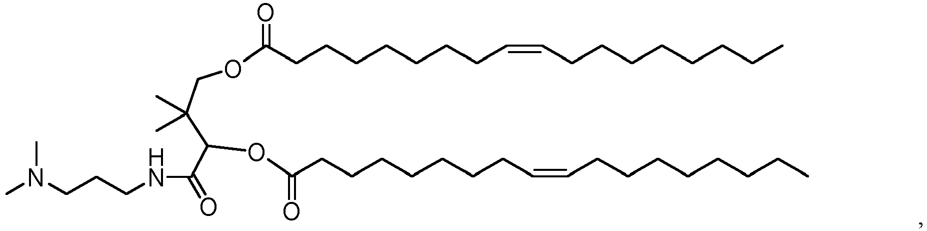

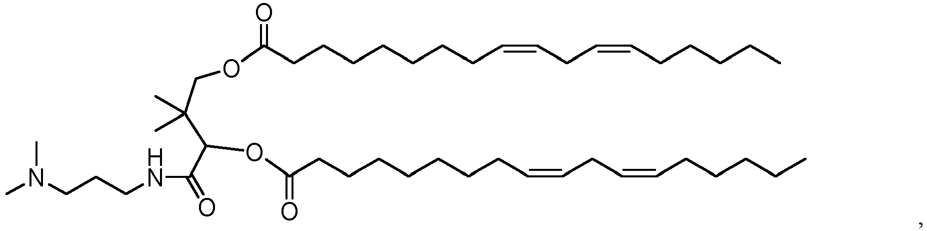

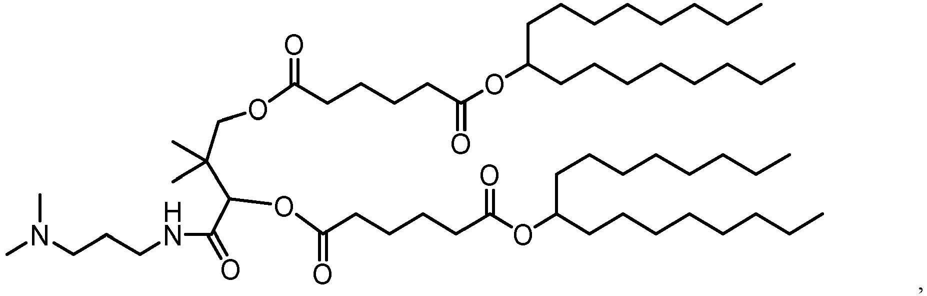

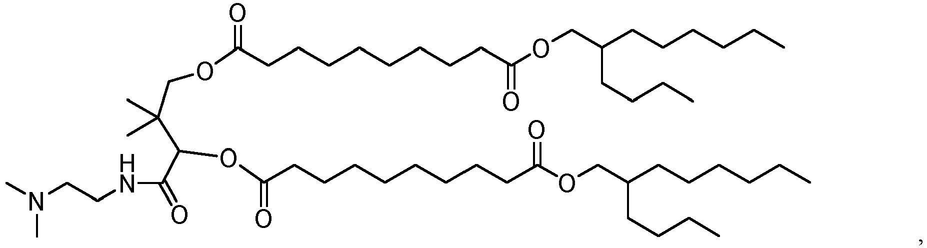

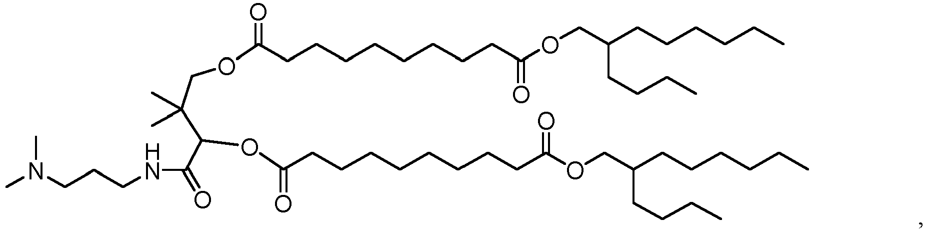

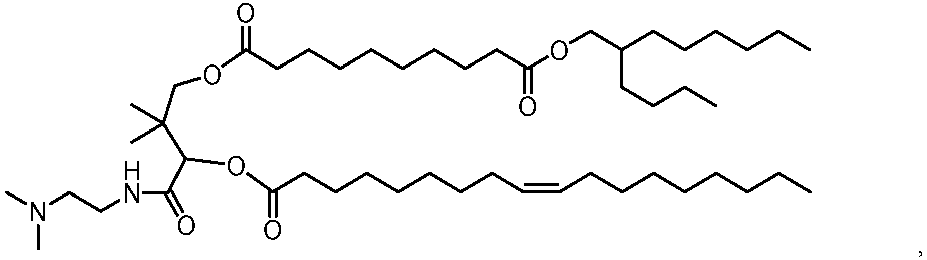

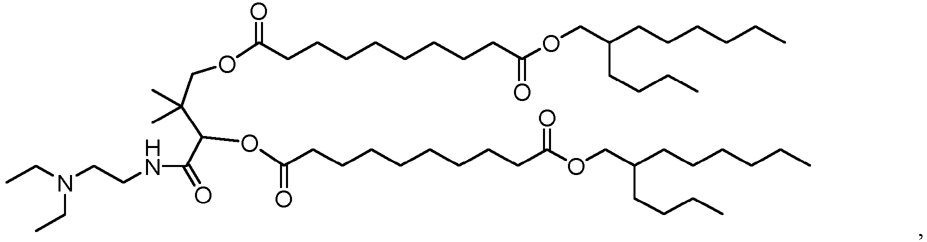

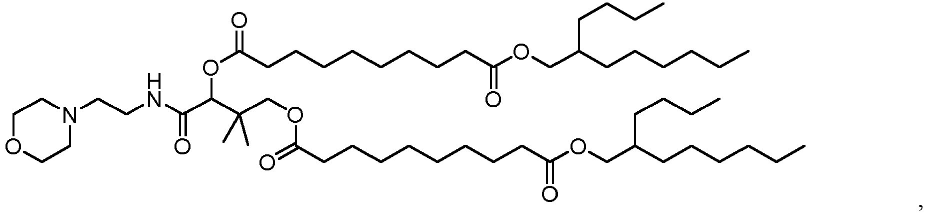

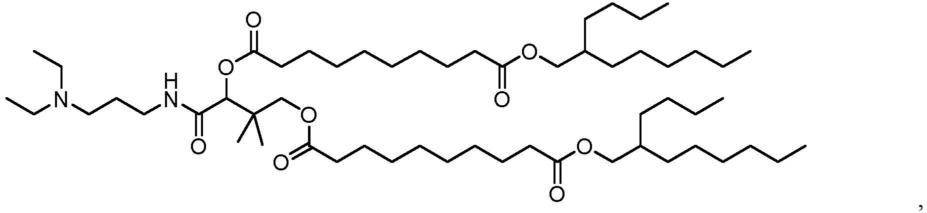

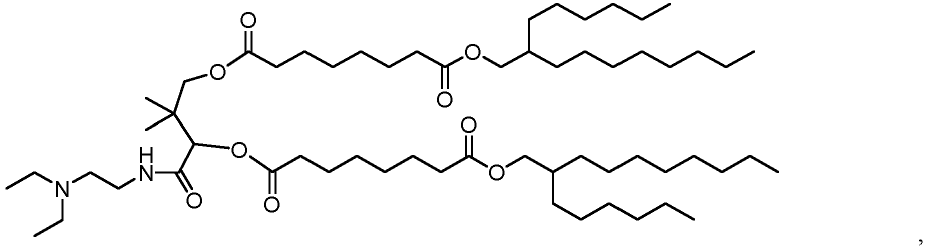

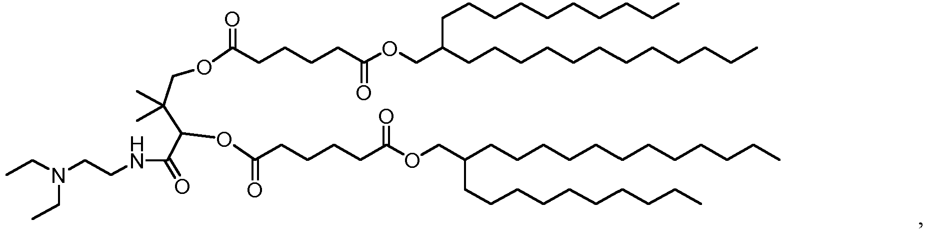

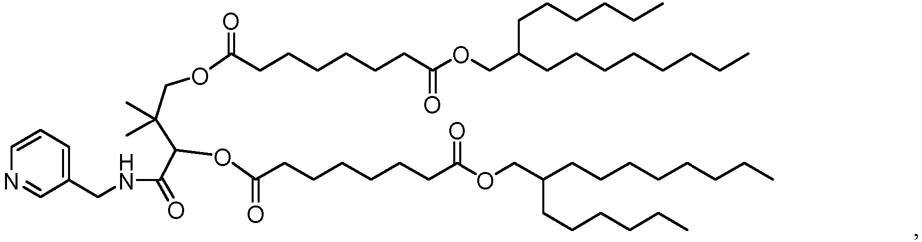

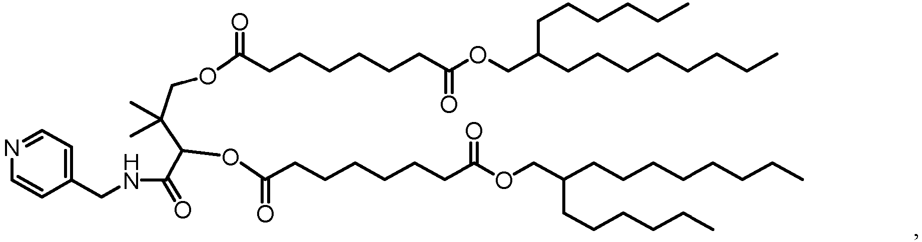

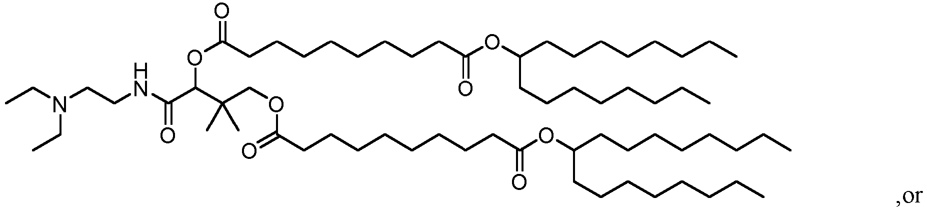

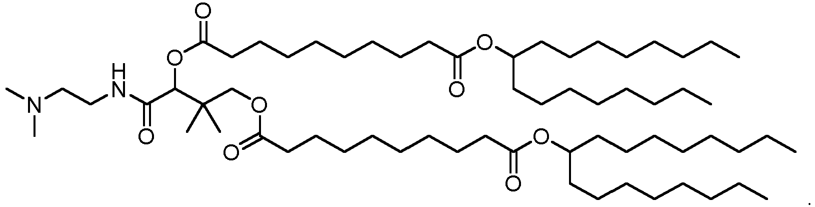

The ionizable lipid of the present disclosure having the above structure can be used to prepare a lipid nanoparticle having a dissociation constant (pKa) of 6.0 to 8.0. Such lipid nanoparticle can be electrically neutral under a neutral physiological condition and positively charged under an acidic condition in a cell, which makes it suitable for use as a lipid nanoparticle carrier.

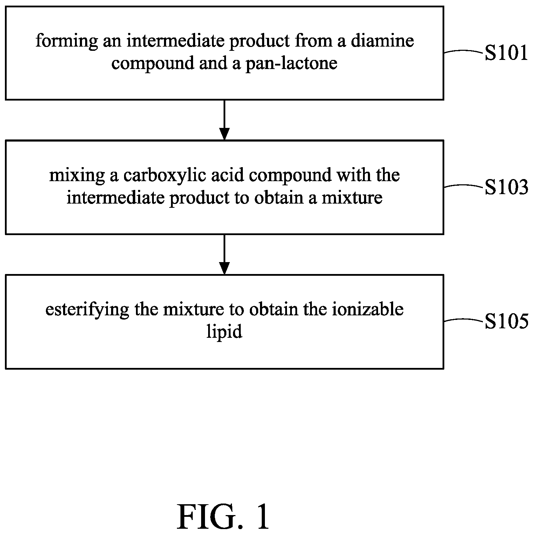

Some embodiments of the present disclosure provide a preparation method of an ionizable lipid, wherein the ionizable lipid may have the structure represented by formula (II) above. FIG. 1 is a flowchart of a preparation method of an ionizable lipid according to some embodiments of the present disclosure. As shown in FIG. 1, the preparation method of the ionizable lipid of the present disclosure includes: a step S101 of forming an intermediate product from a diamine compound and a pan-lactone; a step S103 of mixing a carboxylic acid compound with the intermediate product to obtain a mixture; and a step S105 of esterifying the mixture to obtain the ionizable lipid.

The diamine compound and the pan-lactone undergo a ring opening reaction at step S101 to form an intermediate product having a diol group. In some embodiments, the diamine compound may be a diamine compound including a tertiary amine. In some embodiments, the diamine compound may have a structure represented by the following formula (A): 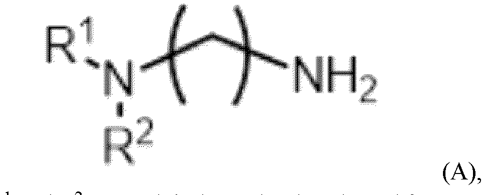 wherein R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group.

In some embodiments, in formula (A), N, R¹ and R² may be bonded to each other to form a heterocycloalkyl group or a heteroaryl group. In some embodiments, the heterocycloalkyl group or the heteroaryl group may further include at least one other heteroatom independently selected from N, O, P, or S. In some embodiments, examples of the diamine compound include 3-(dimethylamino) propylamine, N,N-dimethylethylenediamine, N,N-diethylethylenediamine, *N,N*-diethyl-1,3-propanediamine, 4-(2-aminoethyl)morpholine, 3-methylpyridinamine, 4-methylpyridinamine, and combinations thereof, but the present disclosure is not limited thereto.

The intermediate product formed in step S101 may have a structure represented by the following formula (B): 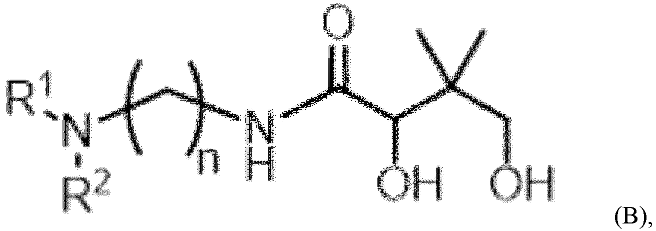 wherein R¹ and R² are the same as those shown in formula (A) above and are not repeated herein.

The intermediate product formed in step S101 is mixed with the carboxylic acid compound in step S103 to form a mixture. In some embodiments, the carboxylic acid compound may have a structure represented by the following formula (C): 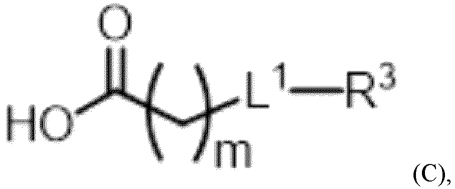 wherein L¹ is independently selected from a group consisting of a C₁-C₁₀ alkylene group, a C₂-C₁₀ alkenylene group, 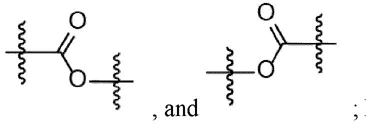 R³ is a C₅-C₃₀ alkyl group; and m is an integer independently selected from 1 to 20.

In some embodiments, L¹ may be independently a C₂-C₁₀ alkenylene group or a C₂-C₁₀ alkadienylene group. R³ may be a C₅-C₃₀ linear alkyl group or a C₅-C₃₀ branched alkyl group. In some embodiments, m is independently an integer selected from 5 to 20. In some embodiments, examples of the carboxylic acid compound include an oleic acid, a linoleic acid, and combinations thereof, but the present disclosure is not limited thereto. In some embodiments, the carboxylic acid compound may be prepared from a branched alcohol compound and a dicarboxylic acid compound. Examples of the alcohol compound include heptadecan-9-ol, 2-butyl-1-octanol, and combinations thereof, but the present disclosure is not limited thereto. Examples of the dicarboxylic acid compound include an adipic acid, an octanedioic acid, a sebacic acid, and combinations thereof, but the present disclosure is not limited thereto.

The mixture obtained in step S 103 may undergo an esterification reaction in step S105 to obtain the ionizable lipid. In some embodiments, the esterification reaction may include use of 1-ethyl-(3-dimethylaminopropyl) carbodiimide, but the present disclosure is not limited thereto.

Some embodiments of the present disclosure provide a composition for preparing a lipid nanoparticle. The composition includes 30 to 75 parts by weight of an ionizable lipid; 5 to 20 parts by weight of a phospholipid compound; 19.5 to 60 parts by weight of a sterol compound; and 0.5 to 5 parts by weight of a polyethylene glycol lipid. In some embodiments, the composition for preparing the lipid nanoparticle of the present disclosure may include 30 to 50 parts by weight of the ionizable lipid; 10 to 20 parts by weight of the phospholipid compound; 35 to 60 parts by weight of the sterol compound; and 1 to 3 parts by weight of the polyethylene glycol lipid.

The ionizable lipid in the composition for preparing the lipid nanoparticle of the present disclosure has a structure represented by formula (I), the specific details of which are described above, and therefore will not be repeated herein.

Non-limiting examples of the phospholipid compound in the composition for preparing the lipid nanoparticle of the present disclosure include, but are not limited to, phosphatidylcholines (PC), phosphatidylethanolamines (PE) and combinations thereof.

Non-limiting examples of the phosphatidylcholines include, but are not limited to, hydrogenated egg phosphatidylcholine (HEPC), hydrogenated soy phosphatidylcholine (HSPC), dipalmitoyl phosphatidylcholine (DPPC), distearyloyl phosphatidylcholine (DSPC), diarachidoyl phosphatidylcholine, dimyristoyl phosphatidylcholine (DMPC), egg phosphatidylcholine (EPC), soy phosphatidylcholine (SPC), oleoyl palmitoyl phosphatidylcholine, dioleoyl phosphatidylcholine (DOPC), dipetroselinoyl phosphatidylcholine, palmitoylelaidoyl phosphatidylcholine, palmitoyloleoyl phosphatidylcholine, dilauroyl phosphatidylcholine (DLPC), diundecanoyl phosphatidylcholine, didecanoyl phosphatidylcholine, and dinonanoyl phosphatidylcholine.

Non-limiting examples of the phosphatidylethanolamines include, but are not limited to, hydrogenated egg phosphatidylethanolamine (HEPE), hydrogenated soy phosphatidylethanolamine (HSPE), dipalmitoyl phosphatidylethanolamine (DPPE), distearyloyl phosphatidylethanolamine (DSPE), diarachidoyl phosphatidylethanolamine, dimyristoyl phosphatidylethanolamine (DMPE), egg phosphatidylethanolamine (EPE), soy phosphatidylethanolamine (SPE), oleoyl palmitoyl phosphatidylethanolamine, dioleoyl phosphatidylethanolamine (DOPE), dipetroselinoyl phosphatidylethanolamine, palmitoylelaidoyl phosphatidylethanolamine, palmitoyloleoyl phosphatidylethanolamine, dilauroyl phosphatidylethanolamine (DLPE), diundecanoyl phosphatidylethanolamine, didecanoyl phosphatidylethanolamine, and dinonanoyl phosphatidylethanolamine.

Non-limiting examples of the sterol compound in the composition for preparing the lipid nanoparticle of the present disclosure include, but are not limited to, cholesterol, cholesterol hexasuccinate, ergosterol, lanosterol, and combinations thereof.

Non-limiting examples of the polyethylene glycol lipid in the composition for preparing the lipid nanoparticle of the present disclosure include, but are not limited to, DMG-PEG2000 (dimyristyl glycerol-PEG2000), DPG-PEG2000 (dipalmitoyl glycerol-PEG2000), DSG-PEG2000 (distearoyl glycerol-PEG2000), DMG-PEG5000 (dimyristyl glycerol-PEG5000), DPG-PEG5000 (dipalmitoyl glycerol-PEG5000), and DSG-PEG5000 (distearoyl glycerol-PEG5000).

Specific embodiments are provided below to further illustrate the features and advantages of the present disclosure. However, it should be understood by a person who has the ordinary skill in the art that the present disclosure is not limited to the specific embodiments disclosed below.

Some synthesis examples of the ionizable lipid of the present disclosure are provided below. All intermediate products and final compounds were purified by a column chromatography on a silica gel, and ¹ H NMR spectra thereof were recorded on a Bruker 600 MHz spectrometer. NMR data were collected in CDCl₃ at ambient temperature (25 °C). Chemical shifts are reported in parts per million (ppm) relative to CDCl₃ (7.26). The following abbreviations were used to express the multiplications: s = singlet; d = doublet; t = triplet; and m = multiplet. All compounds were provided as a mixture of all stereoisomers.

The purities of the final compounds in the following synthesis examples were determined by UPLC/CAD using a Thermo Scientific Vanquish Flex liquid chromatography instrument system equipped with a charged aerosol detection (CAD) and a photodiode array (PDA) (Analytical UPLC-column: Thermo Scientific Accucore C30, 2.6 µm, 100x2.1 mm Mobile phase A: acetonitrile with 0.1% TFA, mobile phase B: isopropanol and water with 0.1% TFA). The purity of the final compounds in each synthesis example was confirmed to be over 80%.

### Synthesis Example 1

### Synthesis of an intermediate product 1 (N-(3-(dimethylamino)propyl)-2,4-dihydroxy-3,3-dimethylbutanamide)

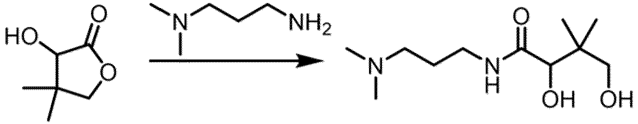

0.78 g (6.0 mmol) of DL-pantolactone and 0.78 mL (7.2 mmol) of 3-(dimethylamino) propylamine were dissolved in 10.0 mL of 1,4-dioxane and stirred under refluxing. The organic solvent was concentrated under reduced pressure. The crude compound was purified by a column chromatography (silica gel, 230 to 400 mesh) using 20% MeOH in DCM solution as an eluent to yield the intermediate product 1 (int 1, 0.97 g, 70% yield) as a white solid. ¹H-NMR (600 MHz, CDCl₃) d 0.92 (s, 3H), 1.01 (s, 3H), 1.70 (m, 2H), 2.24 (s, 6H), 2.37 (m, 2H), 3.36-3.42 (m, 2H), 3.46 (s, 2H), 3.97 (s, 1H)

Synthesis of an ionizable lipid 1 (4-((3-(dimethylamino)propyl)amino)-2,2-dimethyl-4-oxobutane-1,3-diyl dioleate)

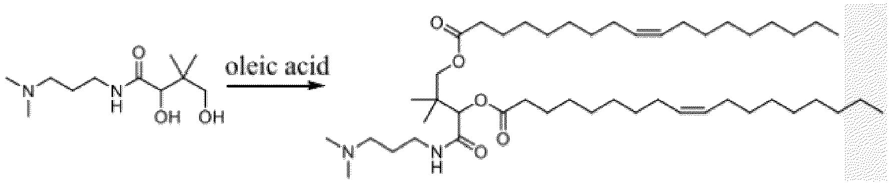

To a mixture of 1.0 g (4.3 mmol) of N-(3-(dimethylamino)propyl)-2,4-dihydroxy-3,3-dimethylbutanamide, 3.0 mL (9.2 mmol) of oleic acid, and 0.51 g (4.2 mmol) of 4-dimethylaminopyridine in DCM (45 mL) was added 2.2 g (10.6 mmol) of N,N'-dicyclohexylcarbodiimide in DCM (45 mL) (4.2 mmol). The resulting mixture was stirred for 4 hours at room temperature. The reaction mixture was filtered by a Celite layer. The filtrate was washed with 50 mL of 1.0 M sodium bicarbonate solution and 40 mL of brine and dried over Na₂SO₄. The organic solvent was removed under reduced pressure. The residue was purified by a column chromatography (silica gel, 230 to 400 mesh) using 0 to 5% MeOH in ethyl acetate as an eluent to yield the ionizable lipid 1 (4-((3-(dimethylamino)propyl)amino)-2,2-dimethyl-4-oxobutane-1,3-diyl dioleate) as a pale yellow oil (2.8 g, 3.6 mmol, 85% yield).
¹H-NMR (600 MHz, CDCl₃) d 0.87 (t, 6H), 1.01 (s, 3H), 1.07 (s, 3H), 1.19-1.37 (m, 40H), 1.57-1.68 (m, 6H), 1.97-2.03 (m, 8H), 2.23 (s, 6H), 2.30 (t, 2H), 2.33-2.43 (m, 4H), 3.23-3.32 (m, 1H), 3.36-3.45 (m, 1H), 3.86 (d, 1H), 4.01 (d, 1H), 4.99 (s, 1H), 5.30-5.36 (m, 4H), 7.44 (t, 1H). HRMS (TOF) (m/z): calc'd for C₄₇H₈₈N₂O₅ [M+H]: 761.6771, found: 761.6786.

### Synthesis Example 2

### Synthesis of an ionizable lipid 2 (4-((3-(dimethylamino)propyl)amino)-2,2-dimethyl-4-oxobutane-1,3-diyl (9Z,9'Z,12Z,12'Z)-bis(octadeca-9,12-dienoate)

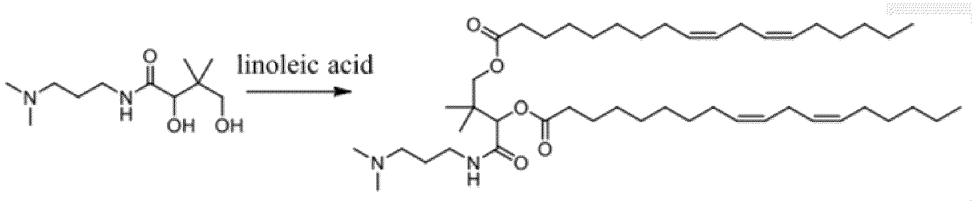

To a solution of N-(3-(dimethylamino)propyl)-2,4-dihydroxy-3,3-dimethylbutanamide (313 mg, 1.35 mmol) and linoleic acid (0.88 ml, 2.85 mmol) in DCM (20 mL) was added 4-dimethylaminopyridine (DMAP, 35 mg, 0.30 mmol) followed by 1-ethyl-3-(3'-dimethylaminopropyl)carbodiimide hydrochloride (620 mg, 3.23 mmol). The resulting mixture was stirred at room temperature for 4 hours, then washed with 50 mL of 1.0 M sodium bicarbonate solution and 40 mL of brine and dried over Na₂SO₄. The organic solvent was removed under reduced pressure. The residue was purified by a column chromatography (silica gel, 230 to 400 mesh) using 0 to 5% MeOH in ethyl acetate as an eluent to yield the ionizable lipid 2 (4-((3-(dimethylamino)propyl)amino)-2,2-dimethyl-4-oxobutane-1,3-diyl (9Z,9'Z,12Z,12'Z)-bis(octadeca-9,12-dienoate) as a pale yellow oil (350 mg, 0.46 mmol, 34% yield).
¹H-NMR (600 MHz, CDCl₃ ) d 0.88 (t, 6H), 1.01 (s, 3H), 1.07 (s, 3H), 1.24-1.41 (m, 28H), 1.55-1.74 (m, 6H), 2.03 (q, 8H), 2.28 (s, 6H), 2.30 (t, 2H), 2.39 (t, 2H), 2.44 (t, 2H), 2.76 (t, 4H), 3.26-3.43 (m, 2H), 3.86 (d, 1H), 4.01 (d, 4H), 3.86 (d, 1H), 4.86 (t, 4H) 2.44 (t, 2H), 2.76 (t, 4H), 3.26-3.43 (m, 2H), 3.86 (d, 1H), 4.01 (d, 1H), 4.97 (s, 1H), 5.29-5.39 (m, 8H), 7.46 (t, 1H), HRMS (TOF) (m/z): calc'd for C₄₇H₈₅N₂O₅ [M+H]: 757.6458, found: 757.6453.

### Synthesis Example 3

### Synthesis of an intermediate product 2 (6-(heptadecane-9-yloxy)-6-oxohexanoic acid)

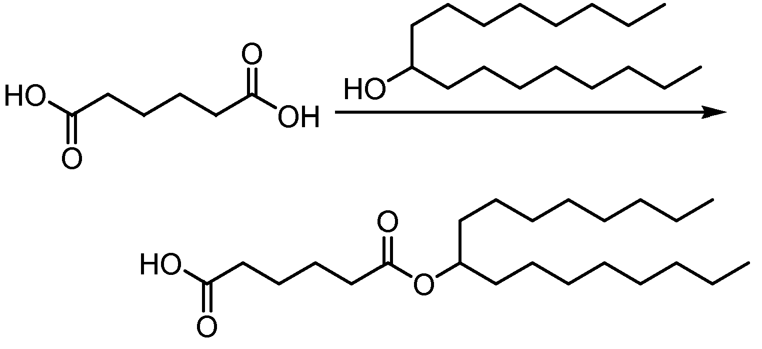

To a solution of adipic acid (3.6 g, 24.6 mmol) and heptadecan-9-ol (2.0 g, 8.2 mmol) in DCM (300 ml) was added DMAP (1.0 g, 8.2 mmol) followed by 1-ethyl-3-(3'-dimethylaminopropyl) carbodiimide hydrochloride (1.57 g, 8.2 mmol). The resulting mixture was stirred at room temperature overnight, then a second portion of DMAP (200 mg, 1.64 mmol) and 1-ethyl-3-(3'-dimethylaminopropyl) carbodiimide hydrochloride (316 mg, 1.64 mmol) was added to the reaction mixture and stirred for another 6 hours at room temperature. The reaction mixture was washed with 1.0 M HCl solution (300 mL) and water (300 mL), and dried over Na₂SO₄. The organic solvent was removed under reduced pressure. The residue was purified by a column chromatography (silica gel, 230 to 400 mesh) using 0 to 5% MeOH in DCM as an eluent to yield the intermediate product 2 (6-(heptadecane-9-yloxy)-6-oxohexanoic acid) as a colorless oil (1.90 g, 61% yield).
¹H-NMR (600 MHz, CDCl₃ ) δ0.86 (t, 6H), 1.18-1.32 (m, 24H), 1.42-1.55 (m, 4H), 1.62-1.72 (m, 4H), 2.30 (t, 2H), 2.36 (t, 2H), 4.86 (m, 1H)

### Synthesis of an ionizable lipid 3 (O,O'-(4-((3-(dimethylamino)propyl)amino)-2,2-dimethyl-4-oxobutane-1,3-diyl) di(heptadecan-9-yl) diadipate)

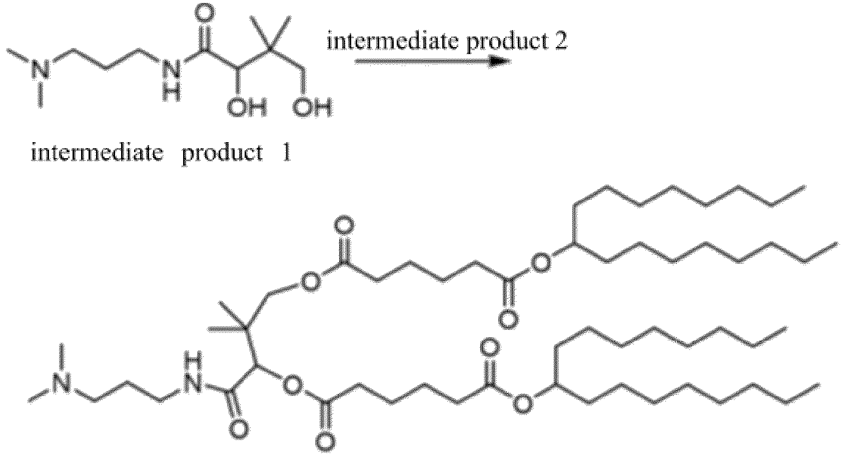

To a solution of 125 mg (0.54 mmol) of the intermediate product 1 and 461 g (1.20 mmol) of the intermediate product 2 in DCM (20 mL) was added DMAP (146 mg, 1.20 mmol) followed by 230 mg (1.20 mmol) of 1-ethyl-3-(3'-dimethylaminopropyl) carbodiimide hydrochloride (230 mg, 1.20 mmol). The resulting mixture was stirred at room temperature for 4 hours. The resulting mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 3 (O,O'-(4-((3-(dimethylamino)propyl)amino)-2,2-dimethyl-4-oxobutane-1,3-diyl) di(heptadecan-9-yl) diadipate) as a pale yellow oil (276 mg, 53% yield).
¹H-NMR (600 MHz, δ0.86 (t, 12H), 1.01 (s, 3H), 1.06 (s, 3H), 1.18-1.31 (m, 48H), 1.42-1.54 (m, 8H), 1.64-1.66 (m, 10H), 2.25 (s, 6H), 2.29-2.41 (m, 10H), 3.25-3.43 (m, 2H), 3.85 (d, 1H), 4.02 (d, 1H), 4.82-4.85 (m, 2H), 4.97 (s, 1H), 7.50 (t, 1H), HRMS (TOF) (m/z): calc'd for C₅₇H₁₀₉N₂O₉ [M+H]: 965.8133, found: 965.8137.

### Synthesis Example 4

### Synthesis of an intermediate product 3 (N-(2-(dimethylamino)ethyl)-2,4-dihydroxy-3,3-dimethylbutanamide)

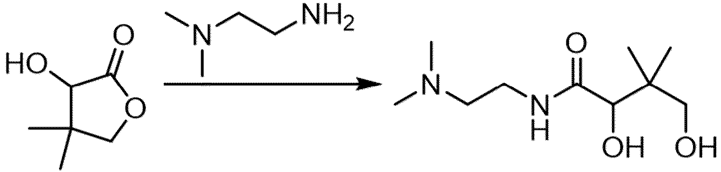

1.5 g (11.5 mmol) of DL-pantolactone and 2.8 mL (25.3 mmol) of N,N-dimethylethylenediamine were dissolved in 25.0 mL of 1,4-dioxane and stirred under refluxing. The organic solvent was concentrated under reduced pressure. The crude compound was purified by a column chromatography (silica gel, 230 to 400 mesh) using a 20% MeOH in ethyl acetate as eluent to yield the intermediate product 3 (int 3) as a yellow solid (1.8 g, 71% yield).
¹H-NMR (600 MHz, δ 0.92 (s, 3H), 1.01 (s, 3H), 2.24 (s, 6H), 2.37 (t, 2H), 3.36-3.42 (m, 2H), 3.45 (s, 2H), 3.97 (s, 1H), 7.25 (s, 1H)

### Synthesis of an intermediate product 4 (10-((2-butyloctyl) oxy)-10-oxodecanoic acid

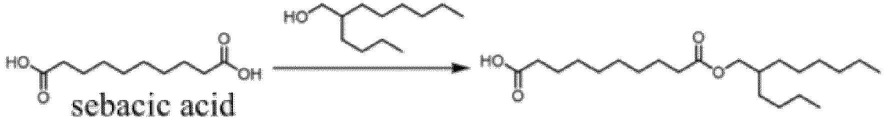

To a solution of 10 g (49.4 mmol) of sebacic acid and 3.6 mL (16.4 mmol) of 2-butyl-1-octanol in DCM (500 ml) was added DMAP (2.0 g, 16.4 mmol), followed by 3.14 g (16.4 mmol) of 1-ethyl-3-((3'-dimethylaminopropyl) carbodiimide hydrochloride. The resulting mixture was stirred overnight at room temperature, then a second portion of DMAP (1.00 g, 8.2 mmol) and 1-ethyl-3-(3'-dimethylaminopropyl) carbodiimide hydrochloride (1.57 g, 8.2 mmol) were added to the reaction mixture and stirred for another 18 hours at room temperature. The reaction mixture was washed with 600 mL of 1.0 M HCl solution and 600 mL of water, and dried over Na₂ SO₄. The organic solvent was removed under reduced pressure. The residue was purified by a column chromatography (silica gel, 230 to 400 mesh) using 5% MeOH in DCM as an eluent to yield the intermediate product 4 (int 4, 10-((2-butyloctyl) oxy)-10-oxodecanoic acid) as a white solid (3.9 g, 64% yield).
¹H-NMR (600 MHz, CDCl₃ ) δ0.84-0.88 (m, 6H), 1.18-1.28 (m, 24H), 1.56-1.64 (m, 5H), 2.27 (t, 2H), 2.31 (t, 2H), 3.95 (d, 2H).

### Synthesis of an ionizable lipid 4 (10-bis(2-butyloctyl)O'¹,O¹-(4-((2-(dimethylamino)ethyl)amino)-2,2-dimethyl-4-oxobutane-1,3-diyl) bis(decanedioate))

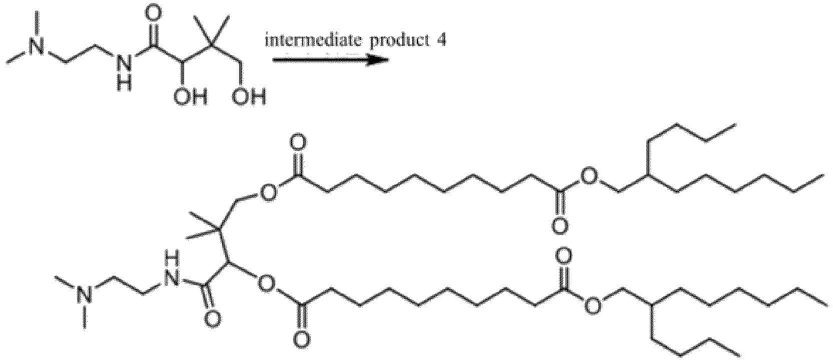

To a solution of 465 mg (2.1 mmol) of the intermediate product 3 and 1.75 g (4.4 mmol) of the intermediate product 4 in DCM (20 mL) was added 530 mg (4.4 mmol) of DMAP, followed by 862 mg (4.4 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by column chromatography (silica gel, 230 to 400 mesh) using 0 to 5% MeOH in DCM as an eluent to yield the ionizable lipid 4 (10-bis(2-butyloctyl)*O*'¹,*O*¹-(4-((2-(dimethylamino)ethyl)amino)-2,2-dimethyl-4-oxobutane-1,3-diyl) bis(decanedioate)) as a pale yellow oil.
¹H-NMR (600 MHz, δ 0.85-0.93 (m, 12H), 1.02 (s, 3H), 1.07 (s, 3H), 1.23-1.36 (m, 48H),1.56-1.68 (m, 10H), 2.19 (s, 6H), 2.26-2.42 (m, 10H), 3.24-3.34 (m, 2H), 3.85 (d, 1H), 3.96 (d, 4H), 4.01 (d, 1H), 4.98 (s, 1H), 6.51 (s, 1H), HRMS (TOF) (m/z): calc'd for C₅₄H₁₀₃N₂O₉ [M+H]: 923.7664, found: 923.7674.

### Synthesis Example 5

### Synthesis of an ionizable lipid 5 (10-bis(2-butyloctyl)O'¹,O¹-(4-((3-(dimethylamino)propyl)amino)-2,2-dimethyl-4-oxobutane-1,3-diyl) bis(decanedioate))

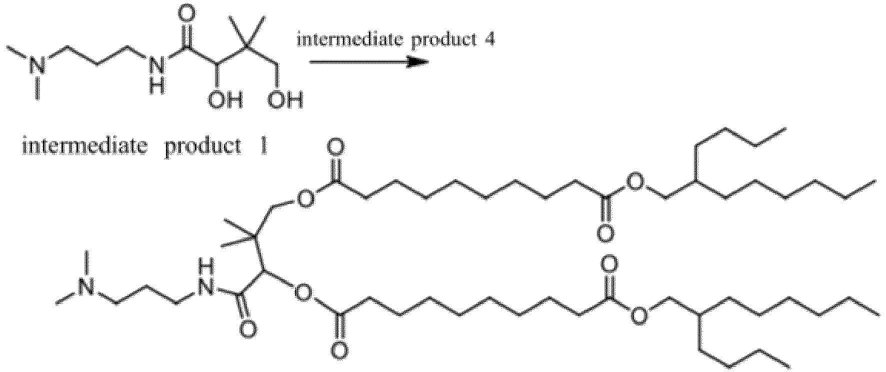

To a solution of 247 mg (1.0 mmol) of the intermediate product 1 and 880 mg (2.2 mmol) of the intermediate product 4 in DCM (20 mL) was added 270 mg (2.2 mmol) of DMAP followed by 420 mg (2.2 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 5 (10-bis(2-butyloctyl)*O*'¹,*O*¹-(4-((3-(dimethylamino)propyl)amino)-2,2-dimethyl-4-oxobutane-1,3-diyl) bis(decanedioate)) as a pale yellow oil (320 mg, 34% yield).
¹ H-NMR (600 MHz, CDCl₃ ) δ 0.85-0.92 (m, 12H), 1.01 (s, 3H), 1.06 (s, 3H), 1.18-1.43 (m, 48H), 1.55-1.66 (m, 10H), 1.67-1.82 (m, 2H), 2.23-2.31 (m, 6H), 2.33 (s , 6H), 2.39 (t, 2H), 2.47-2.57 (m, 2H), 3.27-3.46 (m, 2H), 3.86 (d, 1H), 3.95 (d, 4H), 4.01 (d, 1H), 4.94 (s, 1H), 7.46 (s, 1H), HRMS (TOF) (m/z): calc'd for C₅₅H₁₀₅N₂O₉ [M+H]: 937.7820, found: 937.7816.

### Synthesis Example 6

### Synthesis of an ionizable lipid 6 (1-(2-butyloctyl) 10-(4-((2-(dimethylamino)ethyl)amino)-2,2-dimethyl-3-(oleoyloxy)-4-oxobutyl) decanedioate)

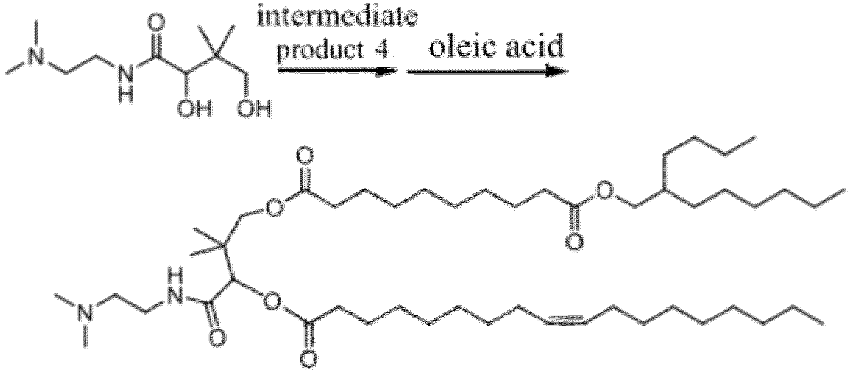

To a solution of 294 mg (1.34 mmol) of the intermediate product 3 and 530 mg (1.34 mmol) of the intermediate product 4 in DCM (30 mL) was added 183 mg (1.50 mmol) of DMAP followed by 287 mg (1.50 mmol) of 1-ethyl-3-(3'-dimethylaminopropyl) carbodiimide hydrochloride. The resulting mixture was stirred at room temperature for 4 hours. The reaction mixture was then concentrated under reduced pressure. The crude mixture was dissolved in 15 mL of DCM and added with 0.42 mL (1.34 mmol) of oleic acid and 183 mg (1.50 mmol) of DMAP. 287 mg (1.50 mmol) of 1-ethyl-3-(3'-dimethylaminopropyl) carbodiimide hydrochloride was then added to the mixture. The reaction mixture was stirred at room temperature for 16 hours, then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 6 (1-(2-butyloctyl) 10-(4-((2-(dimethylamino)ethyl)amino)-2,2-dimethyl-3-(oleoyloxy)-4-oxobutyl) decanedioate) as a pale yellow oil (300 mg, 27% yield). ¹H-NMR (600 MHz, CDCl₃ ) δ 0.85-0.91 (m, 9H), 1.02 (s, 3H), 1.06 (s, 3H), 1.19-1.37 (m, 44H), 1.55-1.69 (m, 7H), 1.96-2.04 (m, 4H), 2.19 (s, 6H), 2.26-2.33 (m, 4H), 2.34-2.42 (m, 4H), 3.24-3.36 (m, 2H), 3.84 (d, 1H), 3.95 (d, 2H), 4.01 (d, 1H), 4.97 (s, 1H), 5.30-5.36 (m, 2H), 6.51 (s, 1H), HRMS (TOF) (m/z): calc'd for C₅₀H₉₅N₂O₇ [M+H]: 835.7139, found: 935.7157.

### Synthesis Example 7

### Synthesis of an intermediate product 5 (N-(2-(diethylamino)ethyl)-2,4-dihydroxy-3,3-dimethylbutanamide)

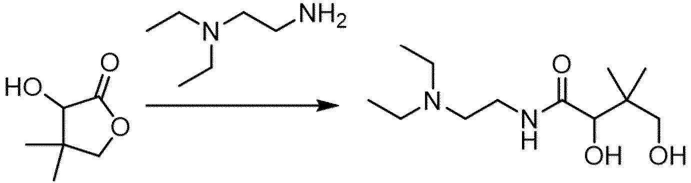

1.5 g (11.5 mmol) of DL-pantolactone and 2.8 mL (25.3 mmol) of N,N-diethylethylenediamine were dissolved in 25.0 mL of 1,4-dioxane and stirred under refluxing. The organic solvent was concentrated under reduced pressure. The crude compound was purified by a column chromatography (silica gel, 230 to 400 mesh) using 20% MeOH in ethyl acetate solution as an eluent to yield the intermediate product 5 (int 5) as a yellow solid (1.8 g, 71% yield).
¹H-NMR (600 MHz, δ0.91 (s, 3H), 0.99 (s, 3H), 1.08 (t, 6H), 2.53-2.58 (m, 6H), 3.32-3.28 (m, 2H), 3.45 (d, 2H), 3.97 (s, 1H)

### Synthesis of an ionizable lipid 7 (10-bis(2-butyloctyl) O'1,O1-(4-((2-(diethylamino)ethyl)amino)-2,2-dimethyl-4-oxobutane-1,3-diyl) bis(decanedioate))

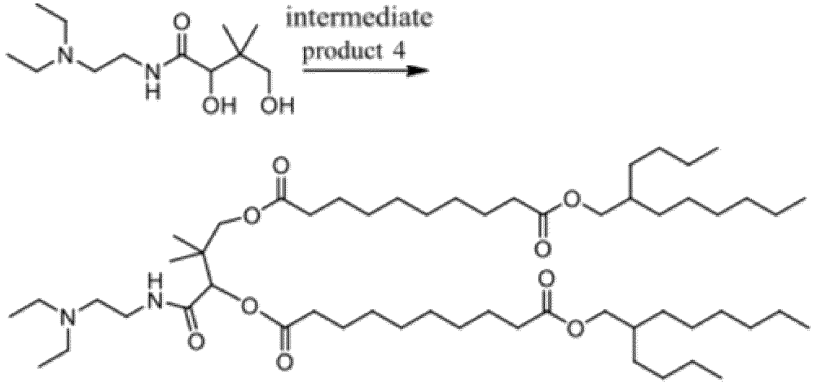

To a solution of 400 mg (1.6 mmol) of the intermediate product 5 and 1.23 g (3.3 mmol) of the intermediate product 4 in DCM (30 mL) was added 430 mg (3.5 mmol) of DMAP, followed by 670 mg (3.5 mmol) of 1-ethyl-3-(3'-dimethylaminopropyl) carbodiimide hydrochloride. The resulting mixture was stirred at room temperature for 4 hours. The reaction mixture was then washed with 30 mL of 1.0 M sodium bicarbonate solution and 30 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 7 (10-bis(2-butyloctyl)*O*'*1*,*O1*-(4-((2-(diethylamino)ethyl)amino)-2,2-dimethyl-4-oxobutane-1,3-diyl) bis(decanedioate)) as a pale yellow oil (720 mg, 47% yield).
¹H-NMR (600 MHz, CDCl₃ ) δ 0.85-0.89 (m, 12H), 0.97-1.04 (m, 9H), 1.09 (s, 3H), 1.21-1.43 (m, 48H), 1.54-1.68 (m, 10H), 2.25-2.33 (m, 6H), 2.34-2.60 (t, 2H), 2.48-2.60 (m, 6H), 3.28-3.30 (m, 2H), 3.86 (d, 4H), 3.86 (d, 4H), 4.01 (d, 2H), 3.96 (d, 4H) 2.48-2.60 (m, 6H), 3.28-3.30 (m, 2H), 3.86 (d, 1H), 3.96 (d, 4H), 4.01 (d, 1H), 5.02 (s, 1H), 6.73 (s, 1H), HRMS (TOF) (m/z): calc'd for C₅₆H₁₀₇N₂O₉ [M+H]: 951.7977, found: 935.7997.

### Synthesis Example 8

### Synthesis of an intermediate product 6 (2,4-dihydroxy-3,3-dimethyl-N-(2-morpholinoethyl)butanamide)

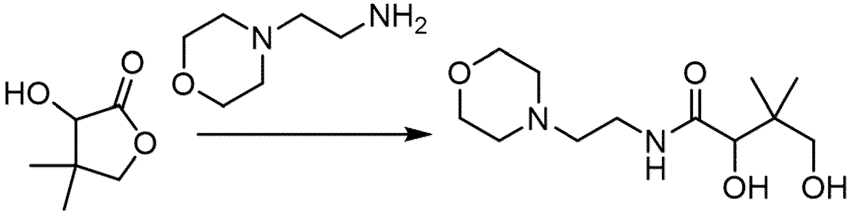

1.1 mL (11.5 mmol) of 4-(2-aminoethyl) morpholine and 1.0 g (11.5 mmol) of DL-pantolactone were mixed in 12.0 mL of 1,4-dioxane and stirred under refluxing for 3 hours. The organic solvent was concentrated under reduced pressure and the crude compound (intermediate product 6 (int 6)) was then directly used for the preparation of an ionizable lipid 8.
¹H-NMR (600 MHz, CDCl₃) δ0.92 (s, 3H), 1.01 (s, 3H), 2.46-2.51 (m, 6H), 3.34-3.51 (m, 4H), 3.69 (t, 3H), 3.99 (s, 1H).

### Synthesis of the ionizable lipid 8 (10-bis(2-butyloctyl)O'1,O1-(2,2-dimethyl-4-((2-morpholinoethyl)amino)-4-oxobutane-1,3-diyl) bis(decanedioate))

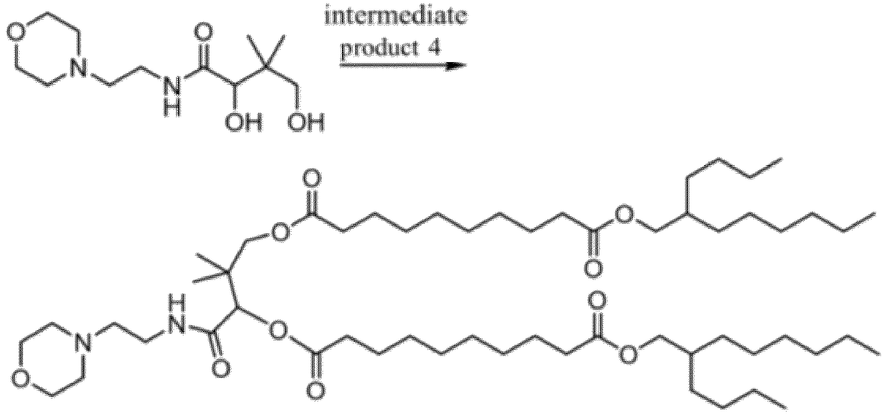

To a solution of 510 mg (1.96 mmol) of diol-intermediate product 6 and 1.45 g (3.92 mmol) of the intermediate product 4 in DCM (30 mL) was added 490 mg (4.0 mmol) of DMAP, followed by 770 mg (4.0 mmol) of 1-ethyl-3-(3'-dimethylaminopropyl) carbodiimide hydrochloride. The resulting mixture was stirred at room temperature for 4 hours. The reaction mixture was then washed with 30 mL of 1.0 M sodium bicarbonate solution and 30 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the colorless ionizable lipid 8 (10-bis(2-butyloctyl)*O*'1,*O*1-(2,2-dimethyl-4-((2-morpholinoethyl)amino)-4-oxobutane-1,3-diyl) bis(decanedioate)) (889 mg, 47% yield).
¹H-NMR (600 MHz, CDCl₃ ) δ0.85-0.87 (m, 12H), 1.021 (s, 3H), 1.065 (s, 3H), 1.21-1.36 (m, 48H), 1.56-1.67 (m, 10H), 2.26-2.49 (m, 14H), 3.18-3.38 (m, 2H), 3.64-3.70 (m, 2H), 3.88 (d, 1H), 3.95 (d, 4H), 4.97 (s, 1H), 4.02 (d, 1H) 3.64-3.70 (m, 2H), 3.88 (d, 1H), 3.95 (d, 4H), 4.02 (d, 1H), 4.97 (s, 1H), 6.40 (t, 1H)

### Synthesis Example 9

### Synthesis of an intermediate product 7 (N-(3-(diethylamino)propyl)-2,4-dihydroxy-3,3-dimethylbutanamide))

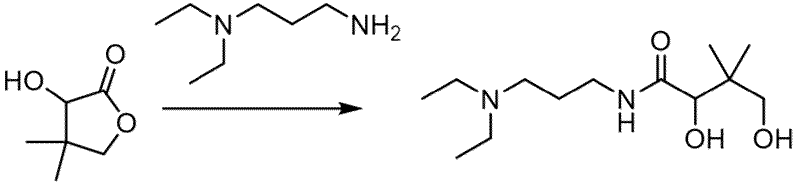

1.3 mL (12.0 mmol) of *N,N*-diethyl-1,3-propanediamine and 1.0 g (11.5 mmol) of DL-pantolactone were mixed in 12.0 mL of 1,4-dioxane and stirred under refluxing for 3 hours. The organic solvent was concentrated under reduced pressure. The crude compound (intermediate product 7 (int 7)) was directly used for next step without purification. ¹H-NMR (600 MHz, CDCl₃ ) *δ* 0.91 (s, 3H), 0.98 (s, 3H), 1.01 (t, 6H), 1.66 (m, 2H), 2.47-2.55 (m, 6H), 3.27-3.39 (m, 2H), 3.43-3.48 (m, 2H), 3.95 (s, 1H), 7.74 (s, 1H). 1H)

### Synthesis of an ionizable lipid 9 (10-bis(2-butyloctyl)O'1,O1-(4-((3-(diethylamino)propyl)amino)-2,2-dimethyl-4-oxobutane-1,3-diyl) bis(decanedioate))

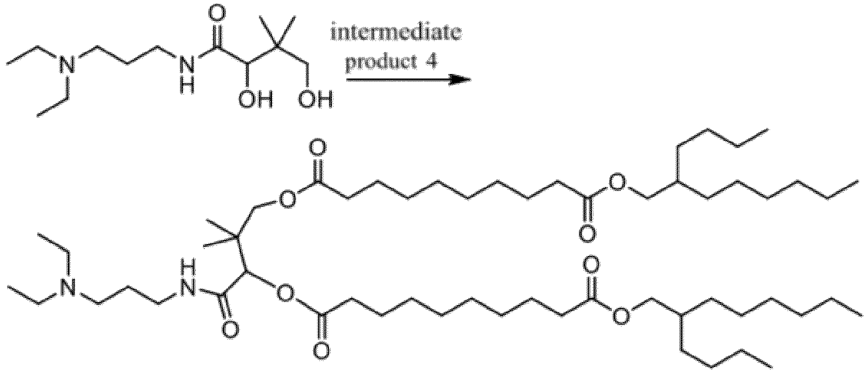

To a solution of 350 mg (1.34 mmol) of diol-intermediate product 7 and 1.00 g (2.7 mmol) of the intermediate product 4 in DCM (20 mL) was added 330 mg (2.7 mmol) of DMAP followed by the 520 mg (2.7 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 9 (10-bis(2-butyloctyl)*O*'1,*O*1-(4-((3-(diethylamino)propyl)amino)-2,2-dimethyl-4-oxobutane-1,3-diyl) bis(decanedioate)) as a colorless oil (543 mg, 42% yield).
¹H-NMR (600 MHz, CDCl₃ ) δ 0.87 (m, 12 H), 1.01 (s, 3H), 1.06-1.08 (m, 9H), 1.25-1.28 (m, 48H), 1.59-1.61 (m, 10H), 1.68-1.71 (m, 2H), 2.26-2.30 (m, 6H), 2.38 (t, 2H), 2.56-2.63 (m, 6H), 3.22-3.28 (m, 1H), 3.41-3.47 (m, 1H), 3.85 (d, 1H), 3.95 (d, 4H), 4.01 (d, 1H), 4.94 (s, 1H), 7.37 (s, 1H)

### Synthesis Example 10

### Synthesis of an intermediate product 8 (8-((2-hexyldecyl)oxy)-8-oxooctanoic acid))

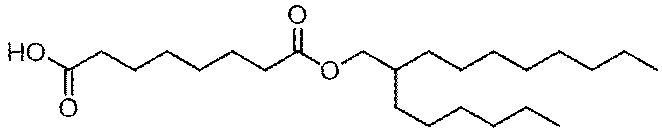

(Intermediate product 8)

8.8 g (64% yield) of the intermediate product 8 (int 8, 8-((2-hexyldecyl)oxy)-8-oxooctanoic acid) was synthesized as a colorless oil from 12.0 g (69.0 mmol) of suberic acid, 10.0 mL (34.5 mmol) of 2-hexyl-1-decanol, 6.6 g (34.5 mmol) of 1-ethyl-3-(3'-dimethylaminopropyl) carbodiimide hydrochloride, and 4.2 g (34.5 mmol) of DMAP in DCM (500 mL) in the same procedure as that for the intermediate product 2.
¹H-NMR (600 MHz, CDCl₃ ) δ 0.87 (t, 6H), 1.18-1.38 (m, 28H), 1.54-1.66 (m, 5H), 2.29 (t, 2H), 2.33 (t, 2H), 3.96 (d, 2H)

### Synthesis of an ionizable lipid 10 (O'1,O1-(4-((2-(diethylamino)ethyl)- amino)-2,2-dimethyl-4-oxobutane-1,3-diyl) 8-bis(2-hexyldecyl) di(octanedioate))

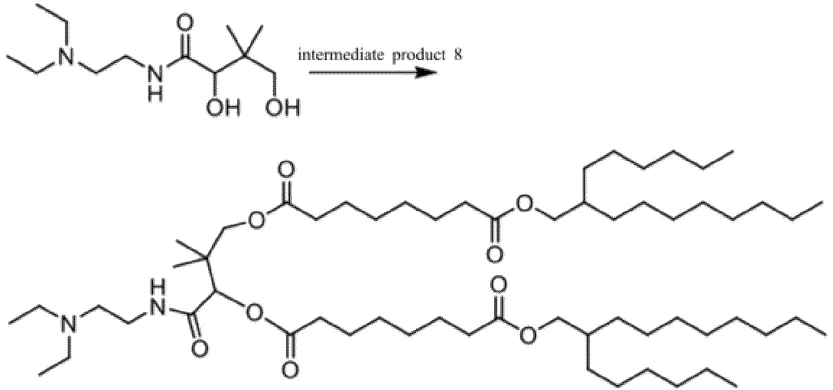

To a solution of 487 mg (1.98 mmol) of diol-intermediate product 5 and 1.60 g (4.0 mmol) of carboxylic acid-intermediate product 8 in DCM (35 mL) was added 488 mg (4.0 mmol) of DMAP, followed by 770 mg (4.0 mmol) of 1-ethyl-3-(3'-dimethylaminopropyl) carbodiimide hydrochloride. The resulting mixture was stirred at room temperature for 4 hours. The reaction mixture was then washed with 35 mL of 1.0 M sodium bicarbonate solution and 35 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 10 (*O'1*, *O1-*(4-((2-(diethylamino)ethyl)- amino)-2,2-dimethyl-4-oxobutane-1,3-diyl) 8-bis(2-hexyldecyl) di(octanedioate)) as colorless oil (937 mg, 47% yield).
¹ H-NMR (600 MHz, CDCl₃ ) *δ* 0.86 (t, 12H), 0.98-1.01(m, 9H), 1.06 (s, 3H), 1.25-1.34(m, 56H), 1.60-1.65 (m,10H), 2.27-2.41(m, 8H), 2.52-2.54(m, 6H), 3.29 (m, 2H), 3.86 (d, 1H), 3.95 (d, 4H), 4.20 (d, 1H), 5.02 (s, 1H), 6.69 (s, 1H) , HRMS (TOF) (m/z): calc'd for C₆₀H₁₁₅N₂O₉ [M+H]: 1007.8603, found: 1007.8618.

### Synthesis Example 11

### Synthesis of an intermediate product 9 (6-((2-decyltetradecyl)oxy)-6-oxohexanoic acid

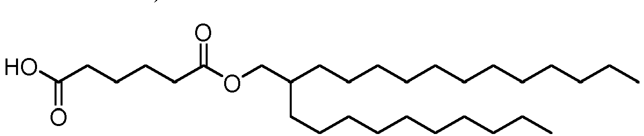

(Intermediate product 9)

The intermediate product 9 (int 9) was synthesized from 5.4 g (36.5 mmol) of adipic acid, 7.0 mL (16.6 mmol) of 2-decyl-1-tetradecanol, 3.2 g (16.6 mmol) of 1-ethyl-3-(3'-dimethylaminopropyl) carbodiimide hydrochloride and 2.1 g (16.6 mmol) of DMAP in DCM (300 mL).
¹ H-NMR (600 MHz, δ0.87 (t, 6H), 1.10-1.40 (m, 40H), 1.61 (s,1H), 1.68 (t, 4H), 2.33 (t, 2H), 2.38 (t, 2H), 3.97 (d, 2H).

### Synthesis of an ionizable lipid 11 (bis(2-decyltetradecyl)O,O'-(4-((2-(diethylamino)ethyl)amino)-2,2-dimethyl-4-oxobutane-1,3-diyl) diadipate)

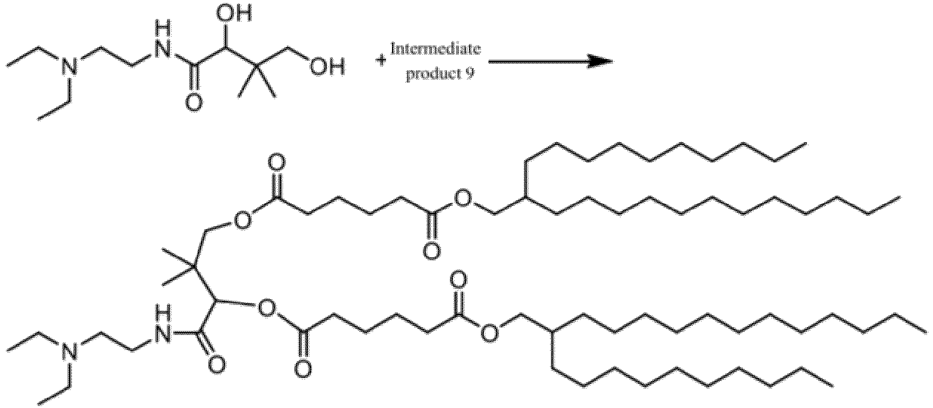

To a solution of 373 mg (1.51 mmol) of diol-intermediate product 5 and 1.46 g (3.0 mmol) of carboxylic acid-intermediate product 9 in DCM (35 mL) was added 370 mg (3.0 mmol) of DMAP, followed by 580 mg (3.0 mmol) of 1-ethyl-3-(3'-dimethylaminopropyl) carbodiimide hydrochloride. The resulting mixture was stirred at room temperature for 4 hours. The reaction mixture was then washed with 35 mL of 1.0 M sodium bicarbonate solution and 35 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 11 (bis(2-decyltetradecyl)O, O'-(4-((2-(diethylamino)ethyl)amino)-2,2-dimethyl-4-oxobutane-1,3-diyl) diadipate) as a colorless oil ( 640 mg, 36% yield).
¹H-NMR (600 MHz, CDCl₃ ) δ 0.87 (t, 3H), 1.01-1.03(m, 9H), 1.07(s, 3H), 1.25-1.30 (m, 80H), 1.65-1.68 (m, 10H), 2.32-2.34 (m, 6H), 2.42-2.43 (m, 2H), 2.55 (m, 6H), 3.31-3.32 (m, 2H), 3.86 (d, 1H), 3.95 (d, 4H), 4.03 (d, 1H), 5.01 (s, 1H), 6.78 (s, 1H), HRMS (TOF) (m/z): calc'd for C₇₂H₁₃₉N₂O₉ [M+H]: 1176.0481, found: 1176.0487.

### Synthesis Example 12

### Synthesis of an intermediate product 10 (2,4-dihydroxy-3,3-dimethyl-N-(pyridin-3-ylmethyl)butanamide)

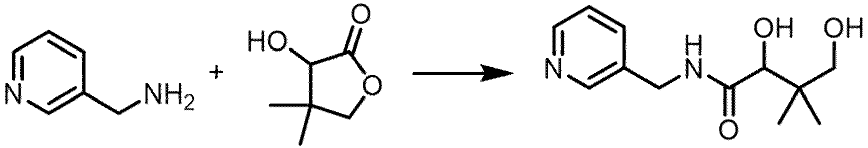

Follow the known procedures of J. Org. Chem. 2023, 88, 14, 10086-10095.

Mix 0.55 mL (5.4 mmol) of 3-picolylamine with 1.30 g (10.0 mmol) of DL-pantolactone, 70.0 mg (0.5 mmol) of 1,5,7-triazabicyclo[4.4.0]dec-5-ene), and 5.0 mL of toluene in a vial and seal in a 20 mL screw vial with a Teflon cap. The mixture was stirred at room temperature for 24 hours. The organic solvent was removed under reduced pressure. The residue was purified by a column chromatography (silica gel, 230 to 400 mesh) using 25% MeOH in DCM as an eluent to yield the intermediate product 10 (int 10, 2,4-dihydroxy-3,3-dimethyl-*N*-(pyridin-3-ylmethyl)butanamide) as a white solid (800 mg, 62% yield).
¹ H-NMR (600 MHz, δ0.92 (s, 6H), 3.38 (d, 1H), 3.47 (d, 1H), 3.96 (s, 1H), 4.44 (s, 2H), 7.37-7.40 (m, 1H), 7.81 (d, 1H), 8.41-8.42 (m, 1H), 8.52 (d, 1H)

### Synthesis of an ionizable lipid 12 (O'1,O1-(2,2-dimethyl-4-oxo-4-((pyridin-3-ylmethyl)amino)butane-1,3-diyl) 8-bis(2-hexyldecyl) di(octanedioate))

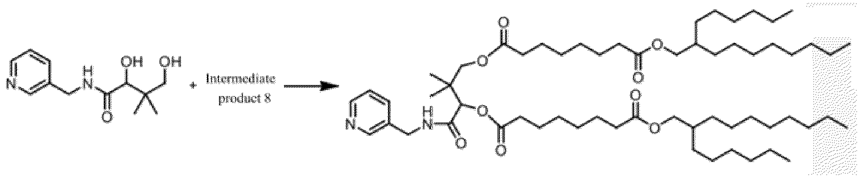

To a solution of 250 mg (1.01 mmol) of diol-intermediate product 10 and 880 mg (2.20 mmol) of carboxylic acid-intermediate product 8 in DCM (20 mL) was added 270 mg (2.2 mmol) ofDMAP followed by 422 mg (2.2 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 12 (*O'1*,*O1-*(2,2-dimethyl-4-oxo-4-((pyridin-3-ylmethyl)amino)butane-1,3-diyl) 8-bis(2-hexyldecyl) di(octanedioate)) as a colorless oil ( 415 mg, 41% yield).
¹H-NMR (600 MHz, CDCl )₃ δ0.87 (s, 12H), 1.01 (s, 3H), 1.05 (s, 3H), 1.24-1.32 (m, 56H), 1.55-1.66 (m, 10H), 2.25-2.37 (m, 8H), 3.85 (d, 1H), 3.93-3.95 (m, 4H), 4.01 (d, 1H), 4.46 (d, 2H), 5.01 (s, 1H), 6.46 (t, 1H), 7.24-7.26 (m, 1H), 7.61 (d, 1H), 8.50-8.51 (m, 2H), HRMS (TOF) (m/z): calc'd for C₆₀H₁₀₇N₂O₉ [M+H]: 999.7977, found: 999.7969.

### Synthesis Example 13

### Synthesis of an intermediate product 11 (2,4-dihydroxy-3,3-dimethyl-N-(pyridin-4-ylmethyl)butanamide)

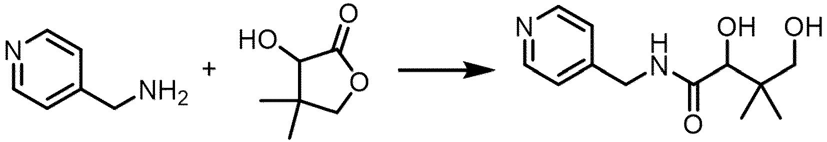

2,4-dihydroxy-3,3-dimethyl-N-(pyridin-4-ylmethyl)butanamide (intermediate product 11, int 11) was synthesized as a colorless oil from 0.55 mL (5.4 mmol) of 4-methylpyridinamine and 1.30 g (10.0 mmol) of DL-pantolactone, 70.0 mg (0.5 mmol) of 1,5,7-triazabicyclo[4.4.0]dec-5-ene, and 5.0 mL of toluene in the same procedure as that for the intermediate product 10.
¹H-NMR (600 MHz, CDCl₃) *δ* 0.95 (s, 6H), 3.40 (d, 1H), 3.49 (d, 1H), 4.0 (s, 1H), 4.46 (m, 2H), 7.38 (d, 2H), 8.46 (d, 2H)

### Synthesis of an ionizable lipid 13 (O'1,O1-(2,2-dimethyl-4-oxo-4-((pyridin-4-ylmethyl)amino)butane-1,3-diyl) 8-bis(2-hexyldecyl) di(octanedioate))

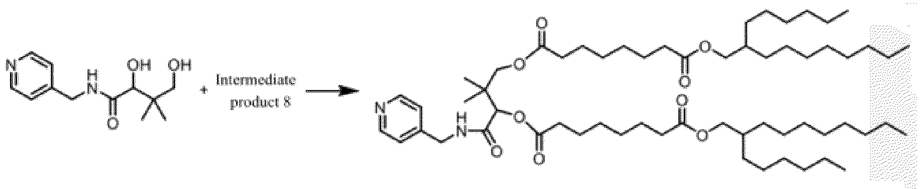

430 mg (43% yield) of the ionizable lipid 13 (*O'1*,*O1*-(2,2-dimethyl-4-oxo-4-((pyridin-4-ylmethyl)amino)butane-1,3-diyl) 8-bis(2-hexyldecyl) di(octanedioate)) was synthesized as a colorless oil from 250 mg (1.01 mmol) of diol-intermediate product 11, 880 mg (2.20 mmol) of carboxylic acid-intermediate product 8, 270 mg (2.2 mmol) of DMAP, and 422 mg (2.2 mmol) of 1-ethyl-3-(3'-dimethylaminopropyl) carbodiimide hydrochloride in DCM (20 mL) in the same procedure as that for the ionizable lipid 12.
¹H-NMR (600 MHz, CDCl₃ ) δ 0.87 (t, 12H), 1.04 (s, 3H), 1.08 (s, 3H), 1.24-1.32 (m, 56H), 1.56-1.64 (m, 10H), 2.26-2.32 (m, 6H), 2.38 (t, 2H), 3.87 (d, 1H), 3.94 (t, 4H), 4.03 (d, 1H), 4.46 (d, 2H), 5.02 (s, 1H), 6.53 (t, 1H), 7.15-7.17 (m, 2H), 8.51-8.54 (m, 2H), HRMS (TOF) (m/z): calc'd for C₆₀H₁₀₇N₂O₉ [M+H]: 999.7977, found: 999.7993.

### Synthesis Example 14

### Synthesis of an intermediate product 12 (10-(heptadecan-9-yloxy)-10-oxodecanoic acid)

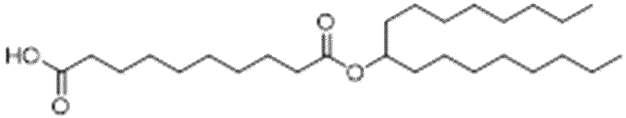

(Intermediate product 12)

9.6 g (66% yield) of the ionizable lipid 12 (int. 12, 10-(heptadecan-9-yloxy)-10-oxodecanoic acid) was synthesized as a colorless oil from 13.4 g (66.2 mmol) of sebacic acid, 8.5 g (33.1mmol) of heptadecane-9-ol, 6.4 g (33.1mmol) of 1-ethyl-3-(3'-dimethylaminopropyl) carbodiimide hydrochloride, and 4.1 g (33.1mmol) of DMAP in DCM (250 mL) in the same procedure as that for the intermediate product 12.
¹H-NMR (600 MHz, CDCl₃ ) *δ* 0.87 (t, 6H), 1.17-1.37 (m, 32H), 1.45-1.53 (m, 4H), 1.56-1.66 (m, 4H), 2.27 (t, 2H), 2.33 (t, 2H), 4.82-4.88 (m, 1H)

### Synthesis of an ionizable lipid 14 (O'1,O1-(4-((2-(diethylamino)ethyl)amino)-2,2-dimethyl-4-oxobutane-1,3-diyl) 10-di(heptadecan-9-yl) bis(decanedioate))

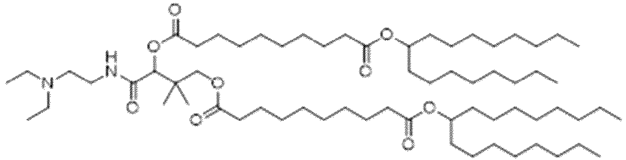

(Ionizable lipid 14)

To a solution of 231 mg (1.1 mmol) of the intermediate product 5 and 1.0 g (2.3 mmol) of the intermediate product 12 in DCM (20 mL) was added 284 mg (2.3 mmol) of DMAP, followed by 460 mg (2.3 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 14 (*O'1*,*O1-*(4-((2-(diethylamino)ethyl)amino)-2,2-dimethyl-4-oxobutane-1,3-diyl) 10-di(heptadecan-9-yl) bis(decanedioate)) as a pale yellow oil (552 mg, 46% yield).
¹H-NMR (600 MHz, CDCl₃ ) δ0.87 (t, 12H), 1.03 (s, 3H), 1.06 (s, 3H), 1.12-1.41 (m, 64H), 1.43-1.54 (m, 8H), 1.55-1.68 (m, 8H), 2.21 (s, 6H), 2.25-2.32 (m, 6H), 2.36-2.41 (m, 2H), 3.23-3.39 (m, 2H), 3.23-4.39 (d, 1H), 4.01 (d, 1H), 4.83-4.39 (m, 2H), 4.01 (d, 1H) 2.36-2.41 (m, 2H), 3.23-3.39 (m, 2H), 3.85 (d, 1H), 4.01 (d, 1H), 4.83-4.87 (m, 2H), 4.98 (s, 1H), 6.55 (s, 1H), HRMS (TOF) (m/z): calc'd for C₆₆H₁₂₇N₂O₉ [M+H]: 1091.9542, found: 1091.9551.

### Synthesis Example 15

### Synthesis of an ionizable lipid 15 (O'1,O1-(4-((2-(dimethylamino)ethyl)amino)-2,2-dimethyl-4-oxobutane-1,3-diyl) 10-di(heptadecan-9-yl) bis(decanedioate))

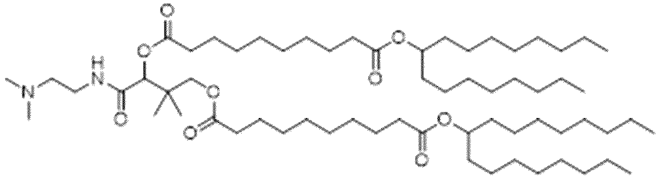

(Ionizable lipid 15)

To a solution of 235 mg (0.95 mmol) of the intermediate product 3 and 924 mg (2.1 mmol) of the intermediate product 12 in DCM (20 mL) was added 260 mg (2.1 mmol) of DMAP, followed by 420 mg (2.1 mmol) of 1-ethyl-3-(3'-dimethylaminopropyl) carbodiimide hydrochloride. The resulting mixture was stirred at room temperature for 4 hours. The reaction mixture was then washed with 20 mL of 1.0 M sodium bicarbonate solution and 20 mL of brine, and dried over Na₂SO₄. The organic solvent was removed under reduced pressure. The residue was purified by a column chromatography (silica gel, 230 to 400 mesh) using 0 to 5% MeOH in DCM as an eluent to yield the ionizable lipid 15 (*O'1*,*O1-*(4-((2-(dimethylamino)ethyl)amino)-2,2-dimethyl-4-oxobutane-1,3-diyl) 10-di(heptadecan-9-yl) bis(decanedioate)) as a pale yellow oil (720 mg, 47% yield).
¹H-NMR (600 MHz, CDCl₃ ) *δ* 0.87 (t, 12H), 0.95 (m, 9H), 1.07 (s, 3H), 1.10-1.42 (m, 64H), 1.44-1.54 (m, 8H), 1.55-1.66 (m, 8H), 2.23-2.32 (m, 6H), 2.39 (t, 2H), 2.54 (s, 6H), 3.30 (d, 2H), 3.86 (d, 1H), 4.02 (d, 1H), 4.83-4.87 (m, 2H), 5.02 (s, 1H), 6.70 (s, 1H), HRMS (TOF) (m/z): calc'd for C₆₄H₁₂₃N₂O₉ [M+H]: 1063.9229, found: 1063.9246.

### Preparation of lipid nanoparticle (LNP) agents A1 to A1 1

An ionizable lipid, DSPC, cholesterol and 1,2-dimyristoyl-sn-glycero-3-phosphoethanolamine-N-[methoxy(polyethylene glycol)-2000] (PEG2k-DMG) were dissolved in pure ethanol in a molar ratio of 50:10:38.5:1.5 according to the following Table 1 to obtain compositions for preparing lipid nanoparticle of the present disclosure. A mRNA solution was prepared using an acidic sodium acetate buffer (pH 4.0 to 5.0) containing mRNAs encoding TriLink luciferase (Luc). The mRNA solution and the composition for preparing lipid nanoparticle of the present disclosure were mixed at a volume ratio of 3:1 using a NanoAssemblr microfluidic system at a 12 mL/min total flow rate to achieve rapid mixing for and self-assembly of the LNP to obtain LNP agents A1 to A11. The LNP agents were further dialyzed against PBS (pH 7.4) and concentrated using centrifugal filtration and filtered (0.2 µm pore size). The particle sizes and polydispersity indexes (PDI) of the LNP agents were measured by dynamic light scattering (DLS) using a Zetasizer Ultra (purchased from Malvern Panalytical). RNA encapsulation efficiencies (EE%) of the compositions for preparing lipid nanoparticle of the present disclosure were determined by Ribogreen assay. The recovery rates of the LNP agents A1 to A11 were measured by Ribogreen. The Zeta Potentials were measured by Zetasizer Ultra (purchased from Malvern Panalytical) using Dynamic Light Scattering (DLS). The acid dissociation constants (pKa) were measured by TNS. The results of each data are shown in Table 1 below.

**Table 1**

| LNP agent No. | Ionizable Lipid | Particle size (nm) | PDI | Encapsulation efficiency (EE, %) | Recovery rate (RR, %) | Zeta Potential (mV) | pKa |
|---|---|---|---|---|---|---|---|
| A1 | Ionizable Lipid 1 | 114.3 | 0.158 | 98.0 | ND | 17.79 | ND |
| A2 | Ionizable Lipid 2 | 184.0 | 0.157 | 97.1 | ND | 13.99 | 8.94 |
| A3 | Ionizable Lipid 3 | 126.4 | 0.024 | 99.5 | 84.5 | -5.69 | 7.78 |
| A4 | Ionizable Lipid 4 | 126.1 | 0.053 | 98.5 | 79.5 | -0.69 | 7.65 |
| A5 | Ionizable Lipid 5 | 289.9 | 0.074 | 97.7 | 35.3 | 2.95 | 8.75 |
| A6 | Ionizable Lipid 8 | 78.4 | 0.015 | 64.9 | ND | -14.99 | 5.49 |
| A7 | Ionizable Lipid 7 | 104.7 | 0.069 | 97.3 | ND | -5.89 | 7.15 |
| A8 | Ionizable Lipid 10 | 101.6 | 0.063 | 98.0 | 94.2 | -4.87 | 7.24 |
| A9 | Ionizable lipid 11 | 108.0 | 0.195 | 98.5 | 82.2 | -5.67 | 6.87 |
| A10 | Ionizable Lipid 14 | 140.6 | 0.164 | 98.7 | 76.0 | -2.70 | 7.27 |
| A11 | Ionizable Lipid 15 | 90.4 | 0.016 | 98.6 | 77.5 | -2.66 | 7.27 |

### Preparation of lipid nanoparticle (LNP) agents B1 to B11

A ionizable lipid, DOPE, cholesterol and 1,2-distearyl-sn-glycero-3-phosphoethanolamine-N-[methoxy(polyethylene glycol)-2000] (PEG2k-DSPE) were dissolved in pure ethanol in a molar ratio of 35:16:46.5:2.5, according to the following Table 2 to obtain the compositions for preparing lipid nanoparticle of the present disclosure. A mRNA solution was prepared using a citric acid buffer (pH 4.0 to 5.0) containing mRNAs encoding TriLink luciferase (Luc). The mRNA solution and the composition for preparing lipid nanoparticle of the present disclosure were mixed at a volume ratio of 3:1 using a NanoAssemblr microfluidic system at a 12 mL/min total flow rate to achieve rapid mixing for and self-assembly of the LNP to obtain LNP agents B1 to B11. The LNP agents were further dialyzed against PBS (pH 7.4) overnight at 4 °C, and concentrated using centrifugal filtration and filtered (0.2 µm pore size). The particle sizes and polydispersity indexes (PDI) of the LNP agents were measured by dynamic light scattering (DLS) using a Zetasizer Ultra (purchased from Malvern Panalytical). RNA encapsulation efficiencies (EE%) of the compositions for preparing lipid nanoparticle of the present disclosure were determined by Ribogreen assay. The recovery rates of the LNP agents B1 to B11 were measured by Ribogreen. The Zeta Potentials were measured by Zetasizer Ultra (purchased from Malvern Panalytical) using Dynamic Light Scattering (DLS). The acid dissociation constants (pKa) were measured by TNS. The results of each data are shown in Table 2 below.

**Table 2**

| LNP agent No. | Ionizable Lipid | Particle size (nm) | PDI | Encapsulation efficiency (EE, %) | Recovery rate (RR, %) | Zeta Potential (mV) | pKa |
|---|---|---|---|---|---|---|---|
| B1 | Ionizable Lipid 1 | 76.1 | 0.203 | 99.0 | ND | 8.96 | 8.01 |
| B2 | Ionizable Lipid 2 | 99.9 | 0.123 | 99.3 | ND | 9.55 | 7.97 |
| B3 | Ionizable Lipid 3 | 133.2 | 0.231 | 99.4 | 84.5 | -5.53 | 6.32 |
| B4 | Ionizable Lipid 4 | 139.5 | 0.237 | 99.2 | 79.5 | 0.51 | 7.83 |
| B5 | Ionizable Lipid 5 | 88.4 | 0.088 | 99.4 | 35.3 | 3.14 | 7.57 |
| B6 | Ionizable Lipid 8 | 83.9 | 0.048 | 94.9 | ND | -10.77 | 5.60 |
| B7 | Ionizable Lipid 7 | 136.3 | 0.228 | 99.1 | ND | -3.65 | 7.38 |
| B8 | Ionizable Lipid 10 | 96.9 | 0.077 | 99.1 | 65.6 | -2.65 | 7.46 |
| B9 | Ionizable lipid 11 | 134.9 | 0.225 | 99.0 | 70.7 | -3.05 | 7.27 |
| B10 | Ionizable Lipid 14 | 94.5 | 0.056 | 99.2 | 69.1 | -0.88 | 6.87 |
| B11 | Ionizable Lipid 15 | 130.4 | 0.229 | 99.0 | 63.0 | -2.31 | 6.89 |

### Preparation of Lipid Nanoparticle (LNP) agents C1 and C2

The LNP agent C1 was prepared in the same manner as the LNP agent A1, except that MC3 (D-Lin-MC3-DMA, purchased from MedChemExpress, the structure thereof is as shown below) was used in place of the ionizable lipid 1 of the present disclosure. The LNP agent C2 was prepared in the same manner as the LNP agent A1, except that SM102 having a structure shown below was used in place of the ionizable lipid 1 of the present disclosure. The particle sizes, polydispersity indexes, encapsulation efficiencies, recovery rates, zeta potentials, and acid dissociation constants of LNP agents C1 and C2 were measured in the same manner as the LNP agent A1. In addition, the solubility of the LNP agents C1 and C2 in ethanol at room temperature was further visually observed. Encapsulated mRNA Concentration was measured by Triton X-100. Cell Viability was measured by One-glo viability. Potency was measured by Luciferase assay. The results of each data are shown in Table 3 below.

### DLin-MC3-DMA

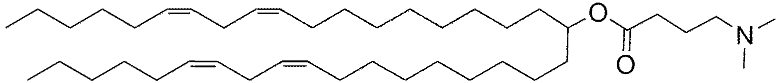

### SM-102

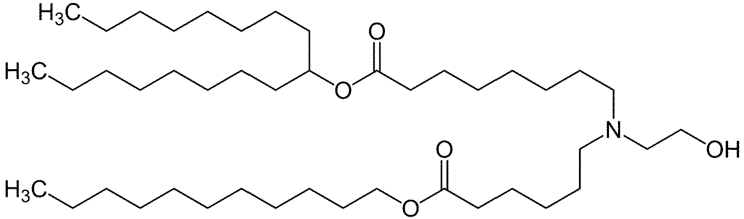

**Table 3**

| LNP agent No. | C1 | C2 |
|---|---|---|
| Ionizable Lipid | MC3 | SM102 |
| Appearance (Visual inspection , Fully soluble in ethanol @ RT) | soluble | soluble |
| Particle Size (DLS, 50-150 nm) | 77.91 | 83.87 |
| PDI (DLS) | 0.047 | 0.04 |
| Zeta Potential (mV) | -8.731 | -5.846 |
| Encapsulation Efficiency (%) (Ribogreen Assay) | 86.4 | 82.9 |
| Recovery Rate (RR, %) | 90.9 | 109.5 |
| Encapsulated mRNA Concentration (mg/mL) (Triton X-100) | 26.2 | 31.3 |
| pKa (TNS Assay) | 6.168 | 6.648 |
| Cell Viability (One-glo viability) | 118.4% | 109.8% |
| Potency (Luciferase assay) | 3.01×10⁷ | 1.16×10⁸ |

From Tables 1 to 3 above, it can be seen that all the LNP agents A1 to A11 and B1 to B11 prepared from the compositions for preparing lipid nanoparticle according to the embodiments of the present disclosure have dissociation constants (pKa) of 6.0 to 8.0. It is apparent that the ionizable lipid of the present disclosure is suitable for the preparation of a lipid nanoparticle for use as a lipid nanoparticle carrier. The polydispersity indexes (PDIs) of the LNP agents A1 to A11 and B1 to B11 are less than 0.3, indicating that the LNP agents A1 to A11 and B1 to B11 are relatively homogeneous.

In addition, the LNP agents A1 to A11 and B1 to B11 have Zeta Potentials of - 30 to 30 mv, which indicated that the lipid nanoparticles in the LNP agents A1 to A11 and B1 to B11 were less likely to interact with the cells, tissues, and other elements in the body, and had a high stability. Further, the absolute values of the Zeta Potentials of LNP agents A3 to A5, A7 to A11, B3 to B5 and B7 to B11 were lower than that of LNP agent C1, indicating that LNP agents A3 to A5, A7 to A11, B3 to B5 and B7 to B11 were more stable than LNP agent C1. The absolute values of the Zeta Potentials of LNP agents A3 to A5, A8 to A11, B3 to B5 and B7 to B11 were lower than that of LNP agent C2, indicating that LNP agents A3 to A5, A8 to A11, B3 to B5 and B7 to B11 were more stable than LNP agent C2. Further, the mRNA encapsulation efficiencies of the LNP agents A1 to A11 and B1 to B11 are higher than those of the LNP agents C1 and C2. That is, the LNP agents prepared from the ionizable lipid of the present disclosure can have a better mRNA delivery effect.

### Preparation of LNP agents D1 to D15

Ionizable lipid 10, DSPC, cholesterol and PEG2k-DMG were dissolved in pure ethanol in a molar ratio shown in the following Table 4 to obtain compositions for preparing lipid nanoparticle of the present disclosure. A mRNA solution was prepared using an acidic sodium acetate buffer (pH 4.0 to 5.0) containing mRNAs encoding luciferase (Luc). The mRNA solution and the composition for preparing lipid nanoparticle of the present disclosure were mixed at a volume ratio of 3:1 using a NanoAssemblr microfluidic system at a 12 mL/min total flow rate to achieve rapid mixing for and self-assembly of the LNP to obtain LNP agents D1 to D15. The LNP agents were further dialyzed against PBS (pH 7.4) and concentrated using centrifugal filtration and filtered (0.2 µm pore size). The particle sizes and polydispersity indexes (PDI) of the LNP agents were measured by dynamic light scattering (DLS) using a Zetasizer Ultra (purchased from Malvern Panalytical). RNA encapsulation efficiencies (EE%) of the compositions for preparing lipid nanoparticle of the present disclosure were determined by Ribogreen assay. The recovery rates of the LNP agents were measured by Ribogreen. The Zeta Potentials were measured by Zetasizer Ultra (purchased from Malvern Panalytical) using Dynamic Light Scattering (DLS). The results of each data are shown in Table 5 below.

**Table 4**

| LNP agent No. | Ionizable lipid 10 | DSPC | cholesterol | PEG2k-DMG |
|---|---|---|---|---|
| D1 | 50 | 10 | 38.5 | 1.5 |
| D2 | 30 | 10 | 59 | 1 |
| D3 | 30 | 15 | 53.5 | 1.5 |
| D4 | 30 | 20 | 48 | 2 |
| D5 | 40 | 5 | 54 | 1 |
| D6 | 40 | 10 | 49.5 | 0.5 |
| D7 | 40 | 15 | 43 | 2 |
| D8 | 40 | 20 | 38.5 | 1.5 |
| D9 | 50 | 5 | 43.5 | 1.5 |
| D10 | 50 | 10 | 38 | 2 |
| D11 | 50 | 15 | 34.5 | 0.5 |
| D12 | 50 | 20 | 29 | 1 |
| D13 | 60 | 10 | 28.5 | 1.5 |
| D14 | 60 | 15 | 24 | 1 |
| D15 | 60 | 20 | 19.5 | 0.5 |

**Table 5**

| LNP agent No. | Particle size (nm) | PDI | Zeta Potential (mV) | Encapsulation efficiency (EE, %) | Recovery rate (RR, %) |
|---|---|---|---|---|---|
| D1 | 106.1 | 0.059 | -6.157 | 98.1 | 88.3 |
| D2 | 100.7 | 0.036 | -3.737 | 97.9 | 87.4 |
| D3 | 84.27 | 0.036 | -12.59 | 98.5 | 90.7 |
| D4 | 76.58 | 0.053 | -6.798 | 98.3 | 93.9 |
| D5 | 113.8 | 0.018 | -5.641 | 97.1 | 81.4 |
| D6 | 118.7 | 0.019 | -11.09 | 93.7 | 77.3 |
| D7 | 78.89 | 0.042 | -4.843 | 98.5 | 95.5 |
| D8 | 82.69 | 0.044 | -5.914 | 97.8 | 83.9 |

| | | | | | |
|---|---|---|---|---|---|
| D9 | 104.7 | 0.035 | -4.181 | 98.7 | 92.7 |
| D10 | 88.32 | 0.056 | -5.005 | 96 | 88 |
| D11 | 111.9 | 0.03 | -6.016 | 96 | 63.9 |
| D12 | 133.6 | 0.207 | -4.426 | 98.1 | 88.8 |
| D13 | 156.5 | 0.055 | -5.108 | 78.8 | 69.3 |
| D14 | 128.5 | 0.038 | -4.632 | 87.3 | 83.3 |
| D15 | 116.4 | 0.052 | -4.577 | 93.7 | 88.1 |

### Cell viability and Potency tests

LNP agents A1 to A4, A6 to A11, C1, and D1 to D15 are tested for their ability to deliver vaccines (or drugs) to cells.

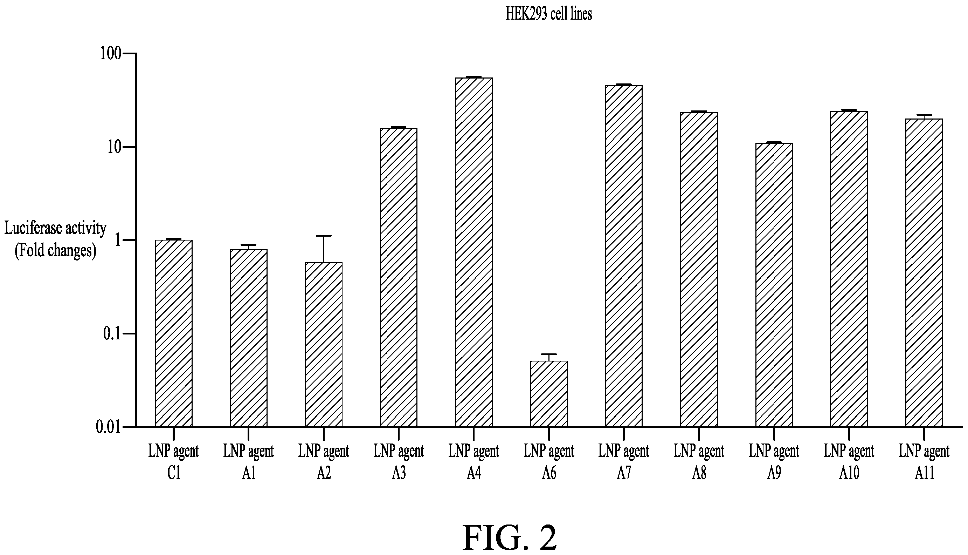

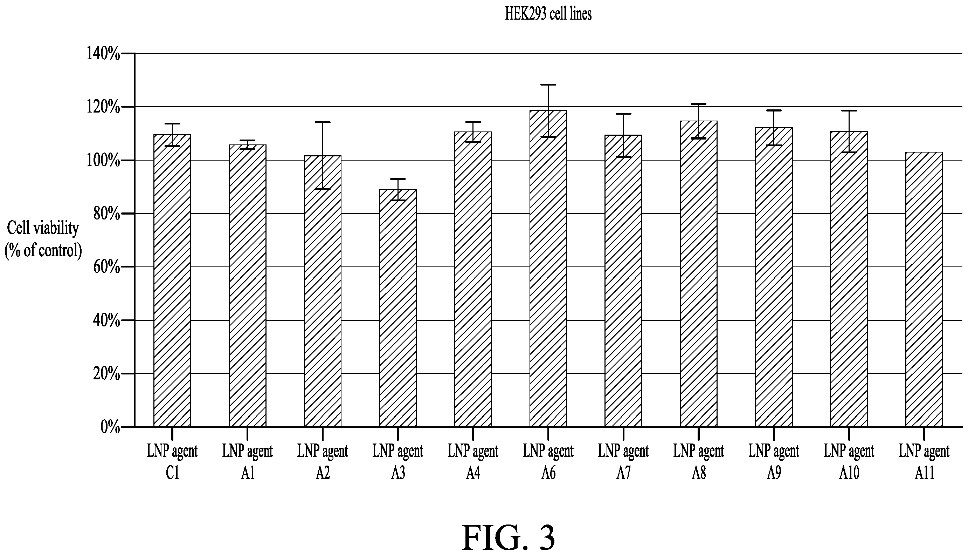

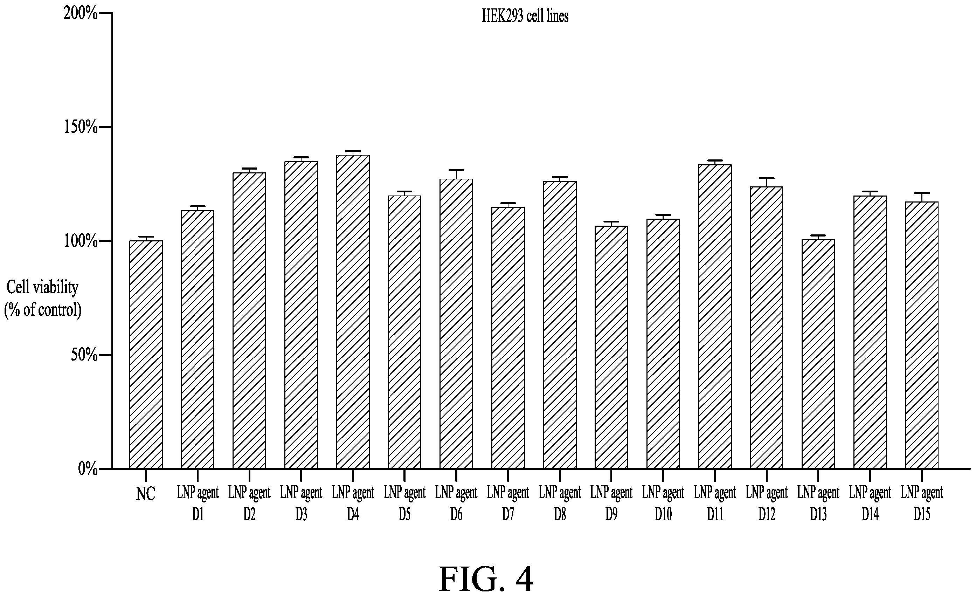

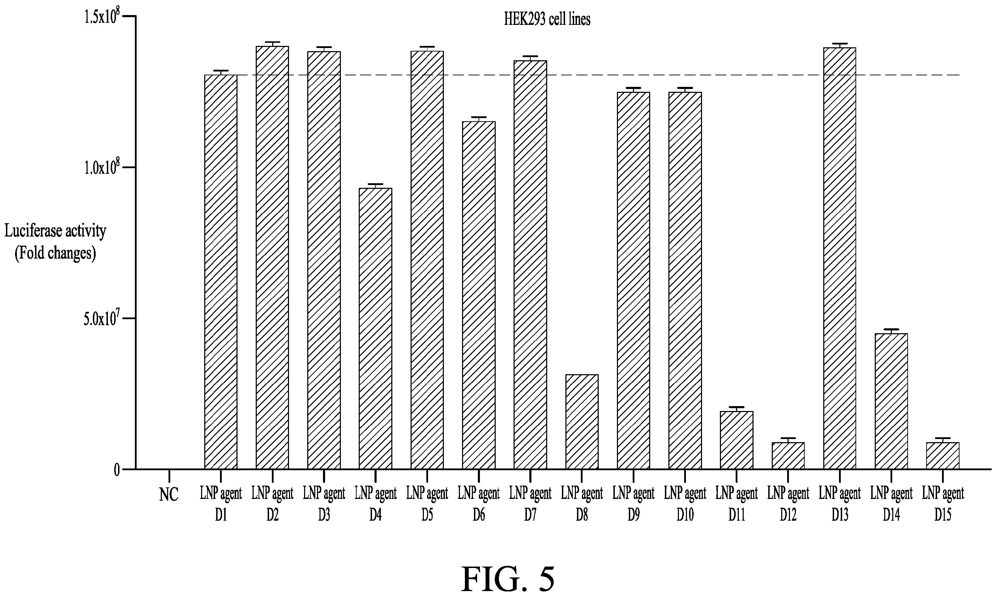

LNP agents A1 to A4, A6 to A11, C1, and D1 to D15 were tested using the ONE-Glo^{™} + Tox Luciferase Reporter and Cell Viability Assay (Promege, E7110). The specific conditions are summarized as follows.
1. Inoculate a 96-well plate with 2*10e4 cell/well of HEK293 cell lines in 100 µL of culture medium one day before the experiment.
2. Replace the culture medium with culture medium containing LNP agents A1 to A4, A6 to A11, C1, and D1 to D15 respectively on the day of the experiment.
3. Add 20 µl of the 5x CellTiter-Fluor^{™} reagent to all wells of the other day and vortex at 400 rpm for 1 minute.
4. Return to incubator at 37°C for 1 hour.
5. Cell viability was obtained by measuring fluorescence 390 nm Ex/505 nm Em using PerkinElmer EnVision Xcite Multilable Reader. The test results are shown in FIG. 3. In FIG. 3, the medium containing only cells and no LNP agent is used as a control group. FIG. 3 is a cell viability graph of HEK293 cell lines treated with the lipid nanoparticle (LNP) agents A1 to A4, A6 to A11, and C1 respectively prepared from the compositions for preparing lipid nanoparticle according to the embodiments of the present disclosure. FIG. 4 is a cell viability graph of HEK293 cell lines treated with the lipid nanoparticle (LNP) agents D1 to D15 respectively prepared from the compositions for preparing lipid nanoparticle according to the embodiments of the present disclosure. In FIG. 4, "NC" refers to a medium containing only cells and no LNP agent. The lower the cell viability is, the higher the toxicity of the LNP agent to the cells.
6. Next, add 100 µl of ONE-Glo^{™} reagent to the wells and incubate for 3 minutes at room temperature.
7. A luciferase activity was measured using PerkinElmer EnVision Xcite Multilable Reader to obtain the potency of the lipid nanoparticle. The results are shown in FIG. 2. FIG. 2 is a graph of luciferase activity of HEK293 cell lines treated with the lipid nanoparticle (LNP) agents A1 to A4, A6 to A11, and C1 respectively prepared from the compositions for preparing lipid nanoparticle according to the embodiments of the present disclosure. FIG. 5 is a graph of luciferase activity of HEK293 cell lines treated with the lipid nanoparticle (LNP) agents D1 to D15 respectively prepared from the compositions for preparing lipid nanoparticle according to the embodiments of the present disclosure. In FIG. 5, "NC" refers to a medium containing only cells and no LNP agent. More luciferase activity indicates higher delivery of the coated material (RNA) and better delivery efficacy of the lipid nanoparticle agent.

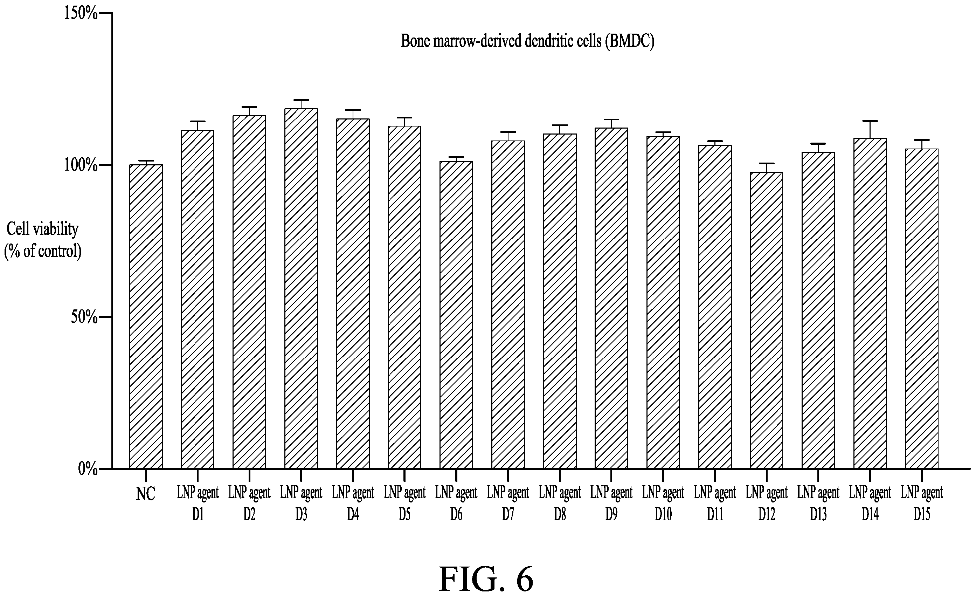

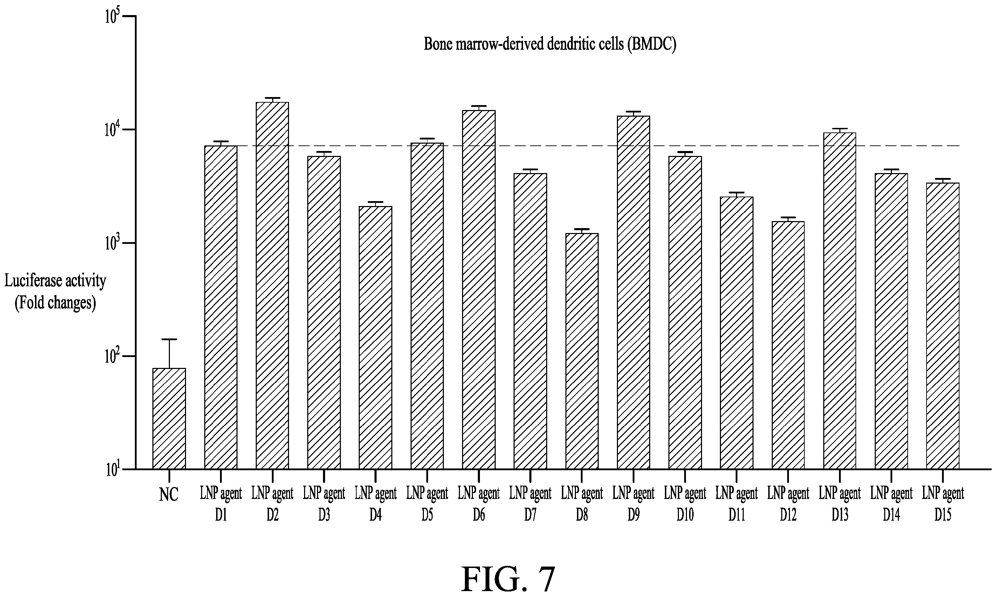

LNP agents D1 to D15 were further tested using the ONE-Glo^{™} + Tox Luciferase Reporter and Cell Viability Assay (Promege, E7110). The specific conditions are summarized as follows.
1. Inoculate a 96-well plate with 2*10e5 cell/well of Bone marrow-derived dendritic cells (BMDC) in 100 µL of culture medium one day before the experiment.
2. Replace the culture medium with culture mediums containing LNP agents D1 to D15 respectively on the day of the experiment.
3. Add 20 µl of the 5x CellTiter-Fluor^{™} reagent to all wells and vortex at 400 rpm for 1 minute.
4. Return to incubator at 37°C for 1 hour.
5. Cell viability was obtained by measuring fluorescence 390 nm Ex/505 nm Em using PerkinElmer EnVision Xcite Multilable Reader. The test results are shown in FIG. 6. FIG. 6 is a cell viability graph of bone marrow-derived dendritic cells (BMDC) treated with the lipid nanoparticle (LNP) agents D1 to D15 respectively prepared from the compositions for preparing lipid nanoparticle according to the embodiments of the present disclosure. In FIG. 6, "NC" refers to a medium containing only cells and no LNP agent. The lower the cell viability is, the higher the toxicity of the LNP agent to the cells.
6. Next, add 100 µl of ONE-Glo^{™} reagent to the wells and incubate for 3 minutes at room temperature.
7. A luciferase activity was measured using PerkinElmer EnVision Xcite Multilable Reader to obtain the potency of the lipid nanoparticle. The results are shown in FIG. 7. FIG. 7 is a graph of luciferase activity of bone marrow-derived dendritic cells (BMDC) treated with one of the lipid nanoparticle (LNP) agents D 1 to D15 respectively prepared from the compositions for preparing lipid nanoparticle according to the embodiments of the present disclosure. In FIG. 7, "NC" refers to a medium containing only cells and no LNP agent. More luciferase activity indicates higher delivery of the payload material (mRNA) and better delivery efficacy of the lipid nanoparticle agent.

As can be seen from FIG. 2, the mRNAs in LNP agents A1 to A4 and A6 to A1 1 can deliver the coated material (RNA) to a cell, indicating that the compositions for preparing lipid nanoparticle according to embodiments of the present disclosure are suitable for preparing lipid nanoparticle that can be used as a vaccine (or drug) delivery carrier for delivering vaccines (or drugs) into a target cell. It can be further seen from FIG. 2 that LNP agents A3, A4, and A7 to A11 have a better ability to deliver vaccines (or drugs) to cells than LNP agent C1. It can be seen from FIG. 3 that the cell viability of the HEK293 cell line treated with LNP agents A1 to A4 or A6 to A11 is more than 80% compared to the control group, indicating that all compositions for preparing lipid nanoparticle according to embodiments of the present disclosure have low toxicity. From FIG. 3, it can be further seen that the LNP agent A4 to A9 have lower toxicity than the LNP agent C 1.

Based on the above experimental results, it can be seen that both the ionizable lipids and the compositions for preparing lipid nanoparticle of the present disclosure can be used to prepare lipid nanoparticles that can be used as vaccine (or drug) delivery carriers for entering vaccines (or drugs) into target cells.

The features of the foregoing embodiments are advantageous to those having ordinary knowledge in the art in understanding the present invention. It should be understood by those of ordinary knowledge in the art that the present invention can be used as a basis for designing and modifying other processes and structures to accomplish the same purpose and/or the same advantages of the above embodiments. It should also be understood by those of ordinary knowledge in the art that these equivalent substitutions are not outside the spirit and scope of the present invention and may be altered, substituted, or changed without departing from the spirit and scope of the present invention.

## Claims

1. An ionizable lipid having a structure represented by the following formula (I): 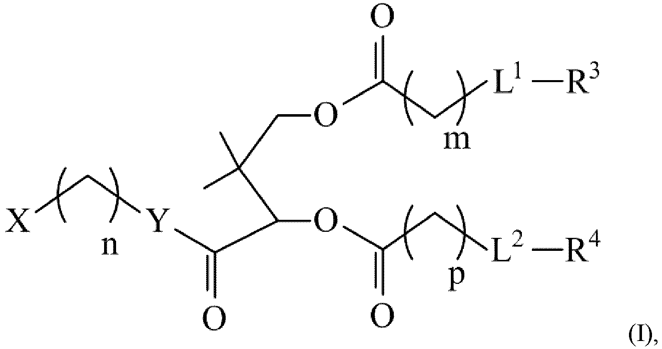
where Y is independently selected from a group consisting of -NH-, -O-, -S-, and a single bond;
X is independently selected from -NR¹R² or a nitrogen-containing heteroaryl group;
L¹ and L² are each independently selected from a group consisting of a C₁-C₁₀ alkylene group, a C₂-C₁₀ alkenylene group, 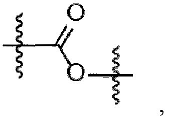 and 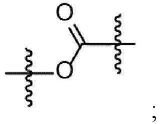 R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer from 1 to 10; and
m and p are each an integer independently selected from 1 to 20.

2. The ionizable lipid as claimed in claim 1, wherein m and p are each an integer independently selected from 5 to 20.

3. The ionizable lipid as claimed in claim 1 or 2, wherein L¹ and L² are each independently a C₂-C₁₀ alkenylene group.

4. The ionizable lipid as claimed in claim 3, wherein L¹ and L² are each independently a C₂-C₁₀ alkadienylene group.

5. The ionizable lipid as claimed in claim 1, having a structure represented by the following formula (I-1): 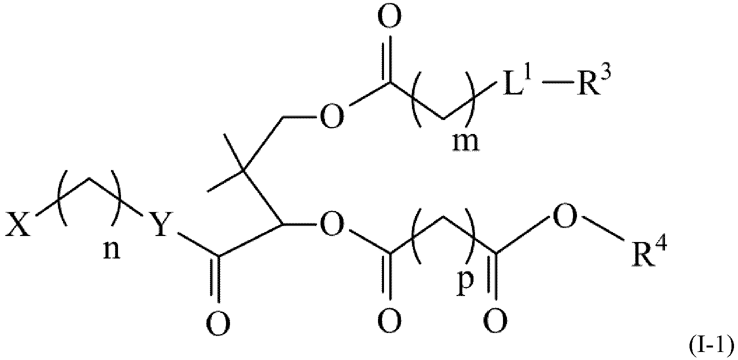 where X, Y, L¹, R³, R⁴, n, m, and p are defined as described in claim 1.

6. The ionizable lipid as claimed in claim 5, wherein L¹ is a C₂-C₁₀ alkenylene group.

7. The ionizable lipid as claimed in claim 5 or 6, wherein R³ is a C₅-C₃₀ linear alkyl group and R⁴ is a C₅-C₃₀ branched alkyl group.

8. The ionizable lipid as claimed in any of claims 5 to 7, wherein m and p are each an integer independently selected from 5 to 20.

9. The ionizable lipid as claimed in any of claims 5 to 8, wherein n is 2.

10. The ionizable lipid as claimed in claim 1, having a structure represented by the following formula (I-2): 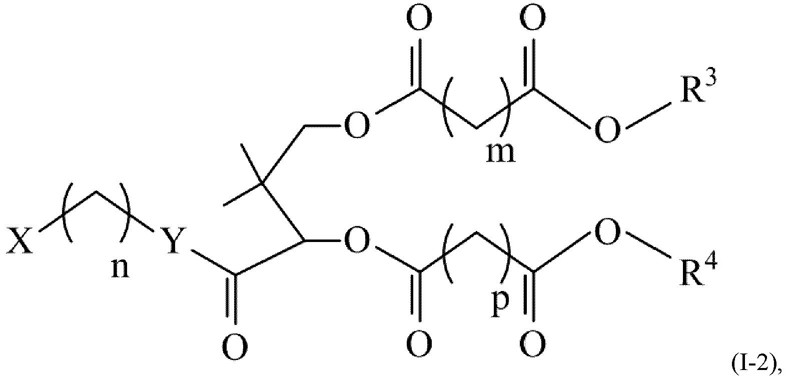 where X, Y, R³, R⁴, n, m, and p are defined as described in Claim 1.

11. The ionizable lipid as claimed in claim 10, wherein m and p are each independently an integer selected from 5 to 20.

12. The ionizable lipid as claimed in claim 10 or 11, wherein n is 2.

13. The ionizable lipid as claimed in any of claims 10 to 12, wherein R³ and R⁴ are each independently a C₅-C₃₀ branched alkyl group.

14. The ionizable lipid as claimed in claim 1, having a structure represented by the following formula (II): 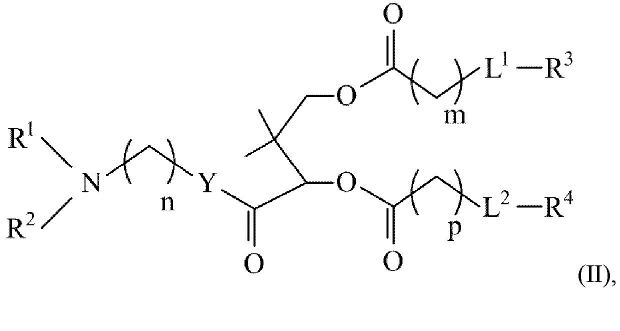 where Y, L¹, L², R¹, R², R³, R⁴, n, m, and p are defined as described in Claim 1.

15. The ionizable lipid as claimed in claim 14, wherein N and R¹ and R² are bonded to each other to form a heterocycloalkyl group or a heteroaryl group.

16. The ionizable lipid as claimed in claim 14 or 15, wherein N and R¹ and R² are bonded to each other to form a heterocycloalkyl group, and the heterocycloalkyl group comprises at least one other heteroatom, and wherein the other heteroatom is independently selected from N, O, P, or S.

17. The ionizable lipid as claimed in claim 1, having a structure represented by the following formula (II-1): 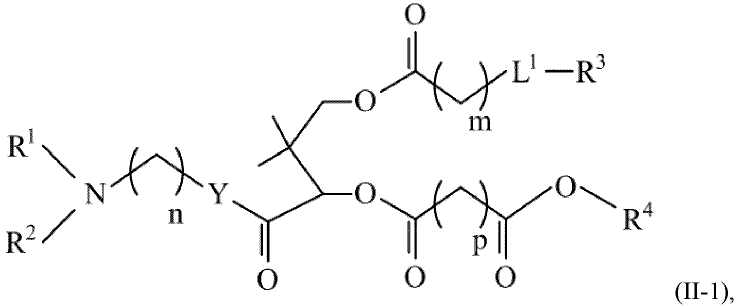 where Y, L¹, R¹, R², R³ , R⁴ , n, m, and p are defined as described in Claim 1.

18. The ionizable lipid as claimed in claim 1, having a structure represented by the following formula (II-2): 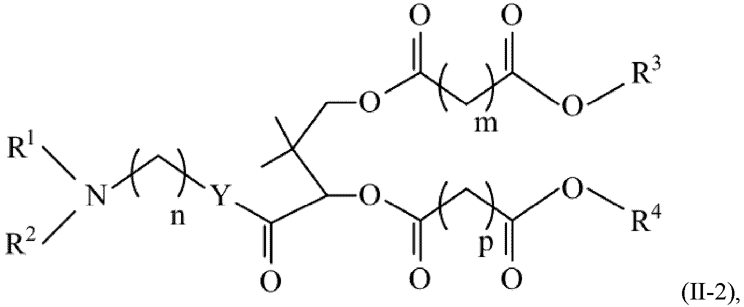 where Y, R¹, R², R³, R⁴, n, m, and p are defined as described in Claim 1.

19. The ionizable lipid as claimed in claim 1, having a structure represented below: 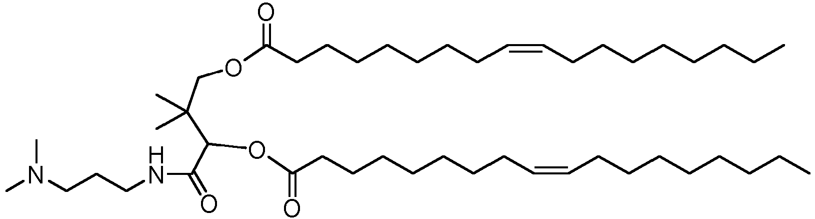 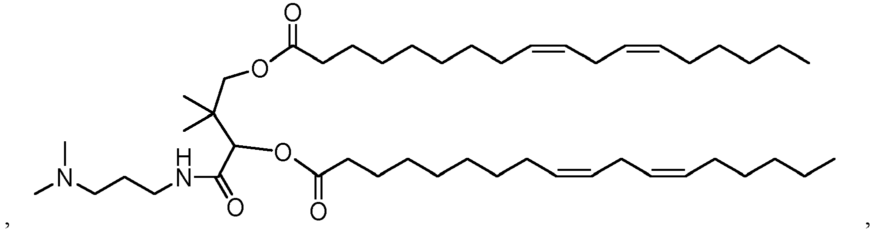 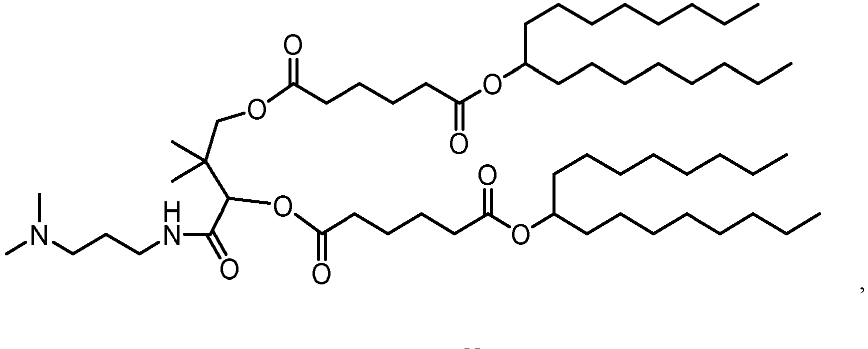 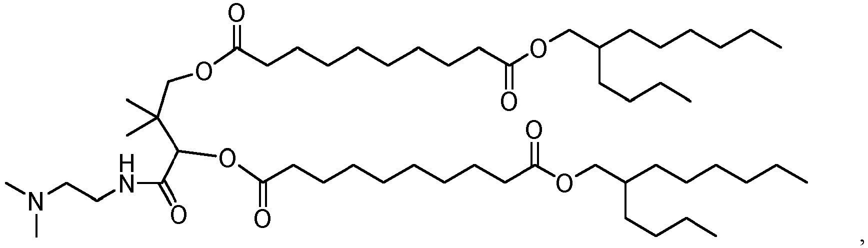 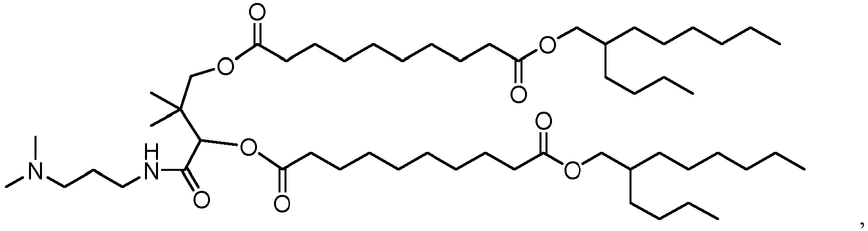 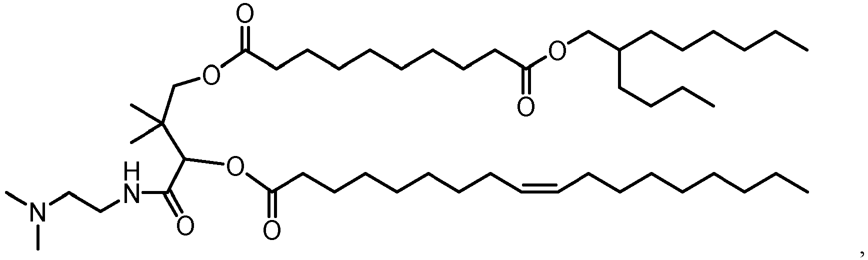 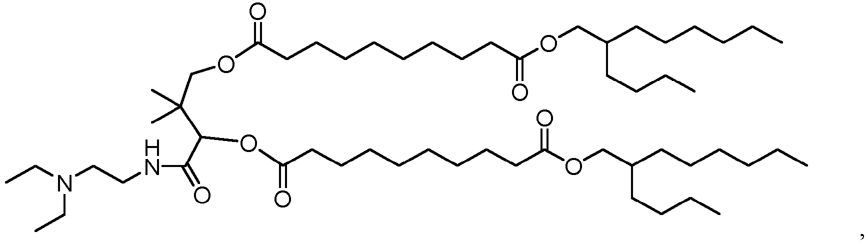 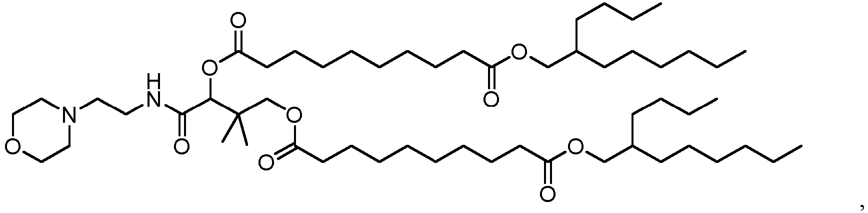 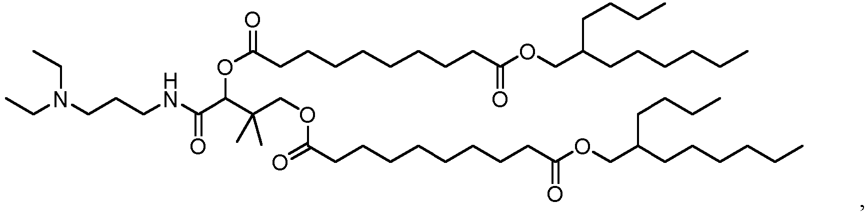 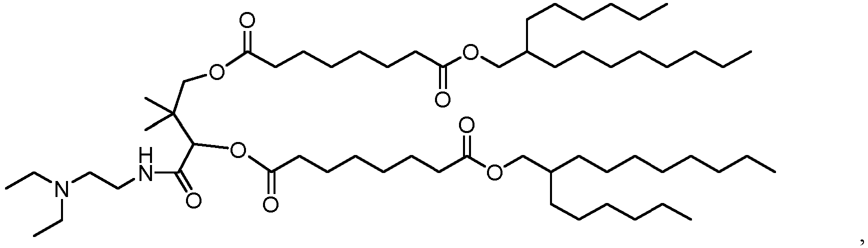 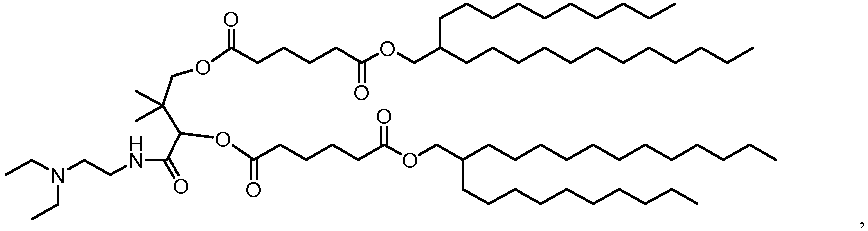 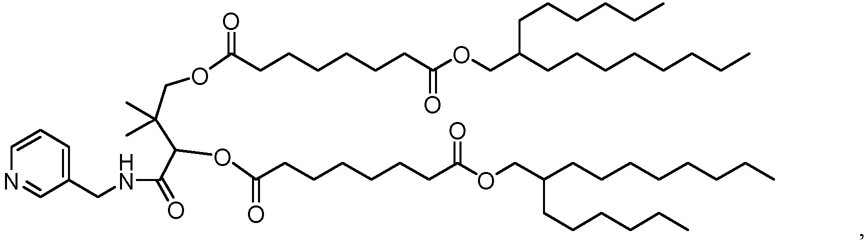 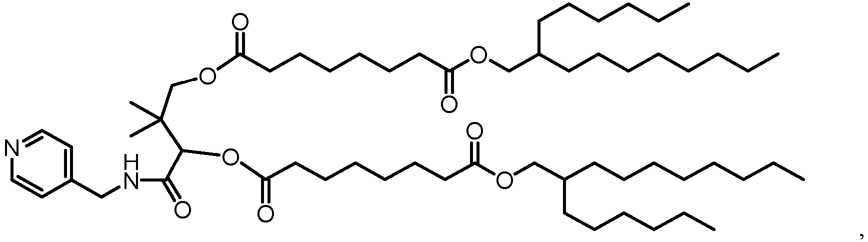 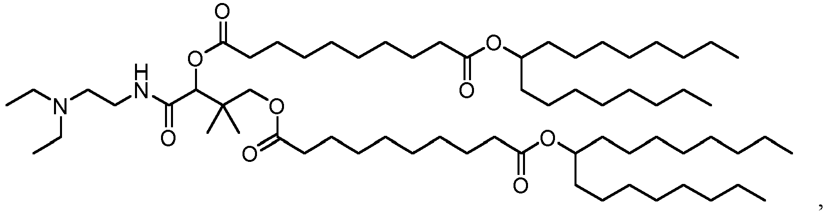 or

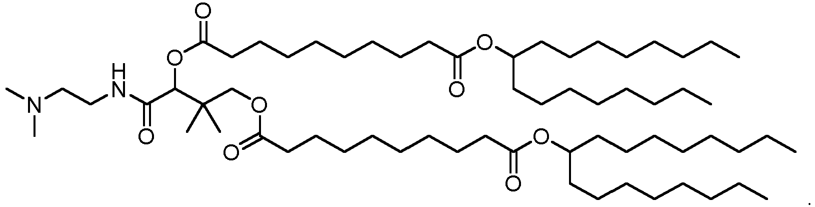

20. A composition for preparing a lipid nanoparticle, comprising:
30 to 75 parts by weight of an ionizable lipid;
5 to 20 parts by weight of a phospholipid compound;
19.5 to 60 parts by weight of a sterol compound; and
0.5 to 5 parts by weight of a polyethylene glycol lipid,
wherein the ionizable lipid has a structure represented by the following formula (I): 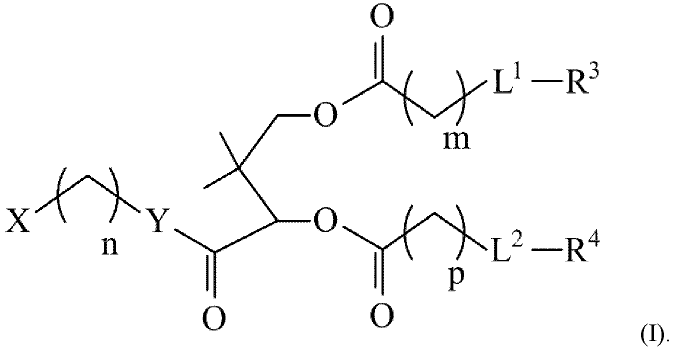
where Y is independently selected from a group consisting of -NH-, -O-, -S-, and a single bond;
X is independently selected from -NR¹R² or a nitrogen-containing heteroaryl group;
L¹ and L² are each independently selected from a group consisting of a C₁-C₁₀ alkylene group, a C₂-C₁₀ alkenylene group, 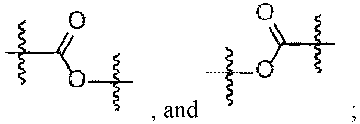
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each independently an integer selected from 1 to 20.

21. A preparation method of an ionizable lipid, comprising:
forming an intermediate product from a diamine compound and a pan-lactone;
mixing a carboxylic acid compound with the intermediate product to obtain a mixture; and
esterifying the mixture to obtain the ionizable lipid,
wherein the ionizable lipid has a structure represented by the following formula (II): 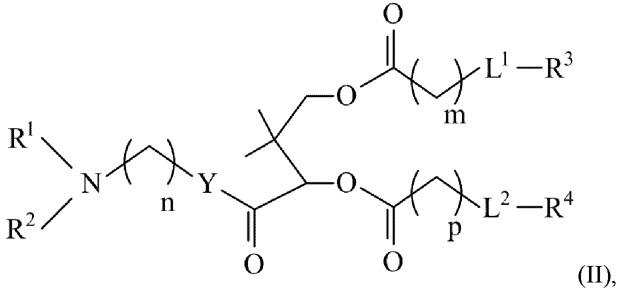
where Y is independently selected from a group consisting of -NH-, -O-, -S-, and a single bond;
L¹ and L² are each independently selected from a group consisting of a C₁-C₁₀ alkylene group, a C₂-C₁₀ alkenylene group, 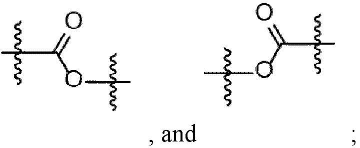
R¹ and R² are each independently selected from a group consisting of H, a substituted or unsubstituted C₁-C₁₀ hydrocarbyl group, a substituted or unsubstituted C₁-C₁₀ heterohydrocarbyl group, a substituted or unsubstituted C₆-C₂₀ aryl group, and a substituted or unsubstituted C₁-C₂₀ heteroaryl group;
R³ is a C₅-C₃₀ alkyl group;
R⁴ is a C₅-C₃₀ alkyl group;
n is an integer selected from 1 to 10; and
m and p are each independently an integer selected from 1 to 20.
